Europäisches Patentamt

European Patent Office

Office européen des brevets

(19)

(11) Publication number: **0 331 100**
**A1**

## (12) EUROPEAN PATENT APPLICATION

(21) Application number: **89103474.6**

(22) Date of filing: **28.02.89**

(51) Int. Cl.⁴: **C12P 21/00** , **G01N 33/577** , **C12N 5/00**

---

The applicant has filed a statement in accordance with Rule 28 (4) EPC (issue of a sample only to an expert). Accession number(s) of the deposit(s): IFO 50168 and FERM BP - 1950

(30) Priority: **02.03.88 JP 47431/88**
**17.06.88 JP 148159/88**
**07.07.88 JP 167733/88**
**18.07.88 JP 177043/88**
**01.11.88 JP 274455/88**

(43) Date of publication of application:
**06.09.89 Bulletin 89/36**

(84) Designated Contracting States:
**AT BE CH DE ES FR GB GR IT LI LU NL SE**

(71) Applicant: **TAKEDA CHEMICAL INDUSTRIES, LTD.**
**3-6, Doshomachi 2-chome Chuo-ku**
**Osaka 541(JP)**

(72) Inventor: **Suzuki, Nobuhiro**
**1-305, 15 Namiki 4-chome**
**Tsukuba Ibaraki 305(JP)**
Inventor: **Kitada, Chieko**
**2-8, Minamikoyocho 1-cho**
**Sakai Osaka 590(JP)**

(74) Representative: **von Kreisler, Alek,**
**Dipl.-Chem. et al**
**Patentanwälte Von Kreisler-Selting-Werner**
**Deichmannhaus am Hauptbahnhof**
**D-5000 Köln 1(DE)**

---

(54) **Anti-endothelin antibodies and their use.**

(57) Polyclonal antibodies and monoclonal antibodies having a specific affinity for endothelin are provided. These antibodies have a high affinity for endothelin and advantageously usable for detection or purification of endothelin or a part thereof by immunological procedures.

EP 0 331 100 A1

## Anti-endothelin Antibodies and Their use

### BACKGROUND OF THE INVENTION

The present invention relates to novel and useful antibodies having specific affinity for endothelin, more particularly to antibodies useful for development of clinical test drugs on the basis of antigen-antibody reactions or for studies of pathologic physiology.

There have been reports of endothelium-dependent vasoconstrictor reactions to various mechanical and chemical stimuli as well as endothelium-dependent vasodilative reactions. However, no endothelium-derived humoral factor mediating these constrictor reactions has yet been identified, except that it has been reported that an antigiotensin is obtained from endothelial cells [Circulation Research, 60, 4221 (1987)]. Recently, Masaki et al. have discovered a novel peptide having a strong vascular smooth muscle constrictor activity and a molecular weight of 2,500 ± 300, which is obtained from mammalian or bird vascular endothelial cells (Japanese patent Application No. 62-255381/1987). One of the isolated and purified peptides from porcine vascular endothelial cells is elucidated to be a peptide consisting of the following 21 amino acid residues (hereinafter referred to as endothelin I) by amino acid analysis (ninhydrin method), molecular weight measurement and other data;

Cys Ser Cys Ser Ser Leu Met Asp Lys glu Cys Val Tyr Phe Cys His Leu Asp Lle Ile Trp
and has 2 set of S-S bonds between Cys and Cys. The molecular weight thereof is 2,492.

Masaki, Onda et al. have cloned porcine and human genes coding genetic information of endothelin (Japanese Patent Application Nos. 62-275613/1987 and 62-313155/1987). Further, two precursors of the above endothelin I have also been obtained, a peptide comprised of about 40 amino acid residues (hereinafter referred to as endothelin II) and a peptide comprised of about 200 amino acid residues (hereinafter referred to as endothelin III). In this specification, endothelin I, endothelin II and endothelin III are generically called endothelin.

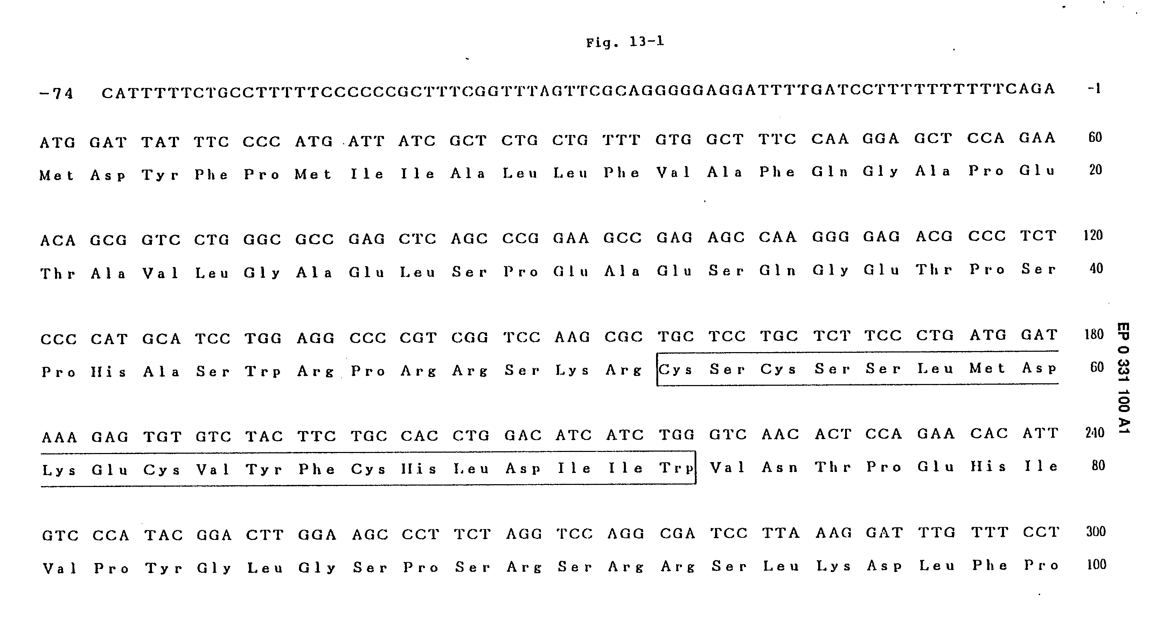

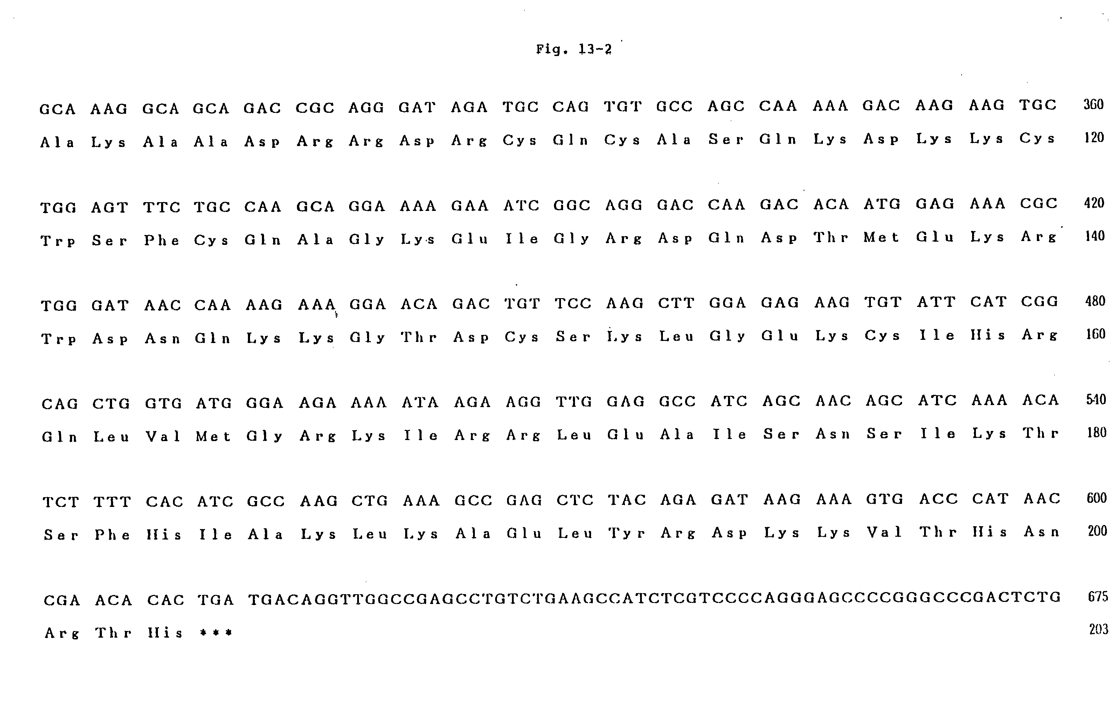

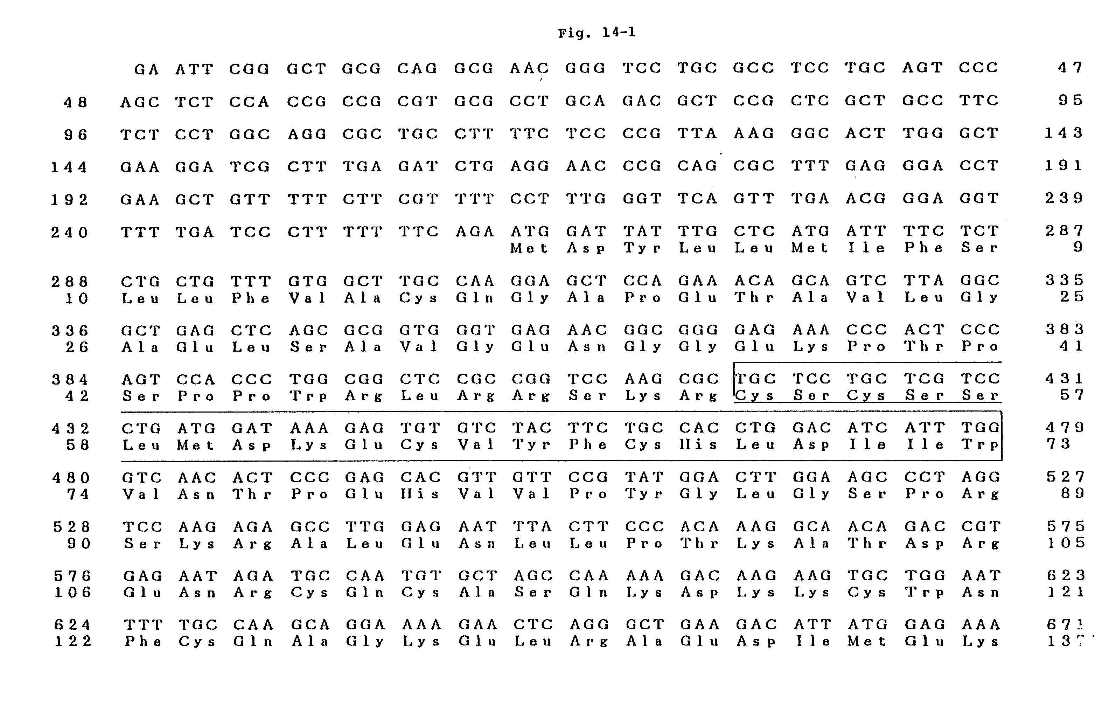

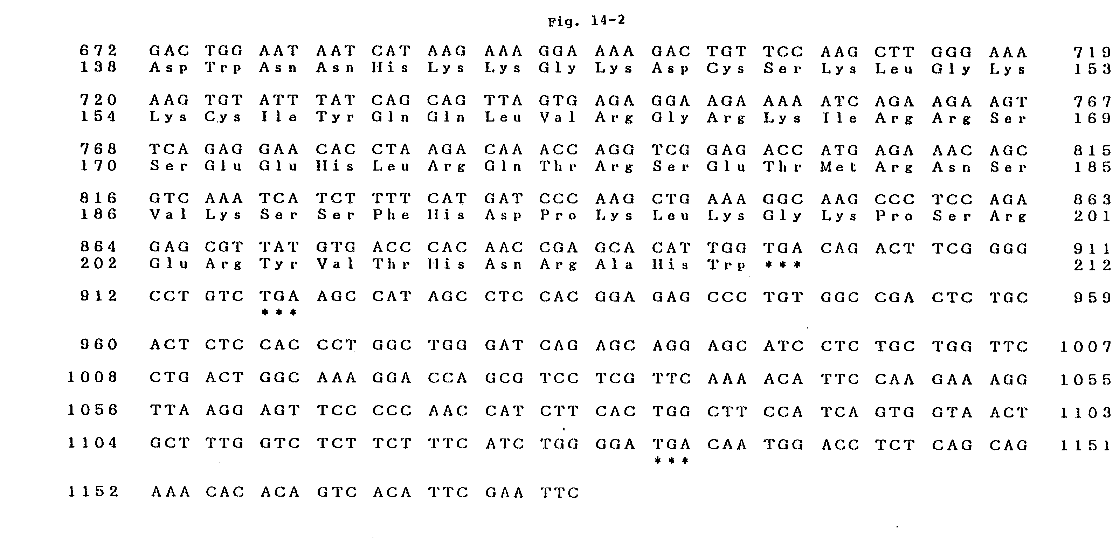

Endothelin II includes, for example, porcine endothelin II comprised of the 53rd to 91st residues in an amino acid sequence shown in Fig. 13-1 and human endothelin II comprised of the 53rd to 90th residues in an amino sequence shown in Fig. 14-1.

Endothelin III includes, for example, porcine endothelin III comprised of the 1st to 203rd residues in the amino acid sequence shown in Fig. 13 and human endothelin III comprised of the 1st to 212th residues in the amino acid sequence shown in Fig. 14.

It has been considered that endothelin might be causally or symptomatically related to various diseases such as essential hypertension and cardiac incompetence, from the viewpoint of its strong vascular smooth muscle constrictor acitivity. For clarifying this point, it is necessary to study endothelin, particularly its metabolic pathways, secretory mechanism, receptors and the like as a whole. Immunological procedures such as immunoassays are considered to be one of the most effective means therefor and it is regarded als highly important to prepare antibodies specific for endothelin. However, the only methods presently available for detecting endothelin are based on biological activity. Therefore, the development of faster, easier and more highly sensitive detecting methods is indispensable for future studies.

### SUMMARY OF THE INVENTION

It is therefore a primary object of the present invention to provide novel antibodies useful for detecting endothelin.

In accordance with the present invention, there are provided polyclonal antibodies or monoclonal antibodies having a specific affinity for endothelin.

### BRIEF DESCRIPTION OF THE DRAWINGS

Fit. 1 is a graph showing antibody titers to immunogen (I) in rabbit antisera;
Fig. 2 is a graph showing antibody titers to immogen (II) in rabbit antisera;
Fig. 3 is a graph showing antibody titers to immogen (III) in rabbit antisera;
Fig. 4 is a graph showing antibody titers to immogen (I) in mouse antisera;

Fig. 5 is a graph showing standard curves of endothelin I, peptide Cys His Leu Asp Ile Ile Trp and angiotensin-II obtained by the competitive-enzyme immunoassay using a polyclonal antibody to immunogen (I);

Fig. 6 is a graph showing standard curves of of endothelin I and peptide Cys His Leu Asp Ile Ile Trp obtained by the competitive method-enzyme immunoassay using a polyclonal antibody to immunogen (II);

Fig. 7 is a graph showing standard curves of of endothelin I and peptide Cys His Leu Asp Ile Ile Trp obtained by the competitive method-enzyme immunoassay using monoclonal antibody AwETN40a;

Fig. 8 is a graph showing standard curve of human endothelin II (-●-), porcine endothelin II (-o-) and human endothelin II C-terminal peptide (-▲-) obtained by the competitive-enzyme immunoassay using a polyclonal antibody to immunogen (III);

Fig. 9 is a graph showing standard curves of of endothelin I obtained by the sandwich method-enzyme immunoassay using monoclonal antibody AwETN40a and anti-endothelin I C-terminal peptide antibody;

Fig. 10 is a graph showing results of experiments conducted in order to heighten sensitivity in Fig. 9 and examine reactivity with endothelin II;

Fig. 11 is a graph showing standard curves of endothelin I obtained by the sandwich method-enzyme immunoassay in which the antibodies are arranged reversely to those in Figs. 9 and 10;

Fig. 12 is a graph showing standard curves of endothelin I and endothelin II obtained by the sandwich method-enzyme immunoassay using monoclonal antibody AwETN40a and anti-endothelin II C-terminal peptide antibody;

Fig. 13 shows an amino acid sequence of porcine endothelin III;

Fig. 14 shows an amino acid sequence of human endothelin III; and

Fig. 15 is a graph showing change of plasma endothelin I of a myocardial infarction patient.

## DESCRIPTION OF THE PREFERRED EMBODIMENTS

The use of the monoclonal antibodies or polyclonal antibodies of the present invention establishes the immunoassay system of endothelin and makes it possible to obtain histochemical information. It is further possible to separate and purify endothelin by immune adsorption chromatography using the anbtibodies of the present invention. These are greatly useful for studying of the physiological significance of endothelin and its relationship to diseases of various kinds, and further for studies and developments of the diagnostics and therapeutic agents therefor.

The polyclonal antibodies of the present invention are usually prepared by producing a conjugate of a carrier protein and endothelin which acts as an immunogen, inoculating animals with this conjugate for immunization, collecting substances containing anti-endothelin antibodies from the immunized animals, and performing separation and purification of the antibodies.

The monoclonal antibodies of the present invention are prepared by selecting individuals having a high antibody titer from the above immunized animals, collecting spleen cells or lymphatic corpuscles therefrom after 3 to 5 days from the final immunization, fusing antibody producing cells containing therein with myeloma cells, and selecting hybridoma cells which stably produce antibody having high titer to obtain monoclonal hybridoma cells.

As endothelin acting as an immunogen, there can be used, for example, natural purified preparations or synthetic preparations described in the foregoing literatures, in which is included the above-mentioned endothelin I, endothelin II, endothelin III and a part thereof.

Various peptides used in the present invention can be prepared by methods known to those skilled in the art for synthesizing peptides, for example, any of the solid phase synthesis methods and liquid phase synthesis methods may be used.

When endothelin is synthesized by the solid phase methods, it is preferable to use the solid phase peptide synthesizing method of Merryfield (J. Am. Chem. Soc., 85, 2149 (1963). There may be used any of the insoluble resins known in the art including, for example, a polystyrene-type resin such as a chloromethylated styrene-divinylbenzene copolymer and a phenacylacetic methylated styrene-divinylbenzene copolymer, and a polyamide-type resin such as a polydimethylacrylamide resin. After a C-terminal N-protected amino acid has been bound to the insoluble resin, protected amino acids are successively combined from the side of the C-terminus of endothelin according to known methods. Then, the resultant product is treated with hydrogen fluoride, followed by the formation of disulfide bonds, whereby the desired endothelin can be synthesized. For N-protected amino acids, all of α-amino acid groups are preferably protected with the Boc group. It is preferred that the hydroxyl groups of serine and threonine are protected with the Bzl groups, the ω-carboxylic acids of glutamic acid and aspartic acid are protected with the OBzl group, the ε-amino group of lysine is protected with the Cl-Z group, the thiol group of cysteine is protected with the Acm group or the MeBzl group, the hydroxyl group of tyrosine is protected with the Br-Z group, the imidazole group of histidine and the guanido group of arginine are protected with the Tos group, and the indole group of tryptophan is protected with the CHO group.

Procedures for synthesizing endothelin in accordance with the liquid phase methods include the methods described in Schroder and Lubke, The Peptides, vol. 1, Academic Press, New York, U.S.A. (1966) or Izumiya et al., Peptide Synthesis, Maruzen (1975), such as the azido method, the chloride method, the acid anhydride method, the mixture anhydride method, the DCC method, the active ester method, the method using Woodward reagent K, the carbodiimidazole method, the redox method and the DCC/additive (for example, HONB, HOBt or HOSu) method.

For the protein conjugates of endothelin and the carrier proteins used for immunizing mammals, any type of carrier protein may be used and the carrier may be added to a hapten (peptide in this case) at any ratio, so long as the antibody can be effectively produced to the hapten coupled with the carrier and immunized. For example, bovine serum albumin, bovine thyroglobulin or hemocyanin is coupled with the hapten at a weight ratio of 0.1 to 20, preferably 1 to 5 per 1 of the hapten.

Various conjugating agents may be used for the coupling of the haptens and the carriers. In particular, glutaraldehyde, a carbodiimide active ester and a maleimide active ester are preferably used.

The conjugated products are administered to warm-blooded animals at sites where the antibody is capable for being produced, by themselves, with a carrier or with a diluent. Particularly, the subcutaneous injection is preferred. In order to enhance the antibody productivity upon the administration, Freund's complete adjuvant or Freund's incomplete adjuvant may be administered. The administration is usually carried out 1 time every 2 to 6 weeks, 3 to 6 times in all.

The warm-blooded animals used therein include, for example, monkey, rabbit, dog, guinea pig, mouse, rat, sheep, goat and chicken.

The antibodies are harvested from the blood, the ascites and the like (preferably the blood) of the warm-blooded animals immunized by the methods described above. The titer of the anti-endothelin antibody in the antiserum is assayed by, for example, reacting labeled endothelin described below with the antiserum, and then measuring the activity of a labeling agent combined with the antibody. The antibody is separated and purified in accordance with the methods known in the art for separating and purifying immunoglobulin. Such methods include salting out, the alcohol precipitation method, the isoelectric precipitation method, the electrophoresis method, the adsorption and desorption method using an ion exchanger (for example, DEAE), the ultracentrifugal method, the gel filtration, and affinity chromatography.

The antibodies thus obtained consist mainly of IgG and contain also additional immunoglobulins such as IgM, IgA and the like. These antibodies are specifically bound to endothelin, but do not coupled with angiotensin-II.

On the other hand, the anti-endothelin antibodies producing hybridoma cells can be prepared by selecting individuals having a high antibody titer from the warm- blooded animals, for example mice, immunized in a similar manner as the foregoing method for preparing the polyclonal antibody, collecting spleens or lymphatic corpuscles therefrom after 2 to 5 days from the final immunization, and fusing antibody producing cells contained therein with myeloma cells. The fusing operation may be conducted according to known methods, for example, the method of Keller and Milstein [Nature, 256, 495 (1975)]. Fusion accelerators include polyethylene glycol (PEG), Sendai virus and the like. In particular, PEG is preferably used. The myeloma cells include, for example, NS-1, P3U1 and SP1.0. Particularly, P3U1 is preferably used. The ratio of the number of the antibody producing cells (lien cells) to the number of the myeloma cells is preferably about 1:1 to 20:1. PEG, preferably PEG 1,000 to PEG 6,000, is added in concentrations of about 10 to 80%, and incubated at 20 to 40°C, preferably 30 to 37°C, for 1 to 10 minutes, whereby the cell fusion is effectively performed.

The anti-endothelin antibody producing hybridoma cells can be screened by various methods known in the art. For example, there is applied an EIA method which comprises adding hybridoma culture supernatant to a solid phase (for example, a microtiter plate) allowed to adsorb endothelin, then adding the anti-immunoglobulin antibody labeled with horseradish peroxidase (HRP) or protein A thereto, and detecting the labelled anti-endothelin monoclonal antibody bound to the solid phase. In this case, when mouse cells are used for the cell fusion, the anti-mouse immunoglobulin antibody is added. There can also be used a method which comprises adding hybridoma culture supernatant to a solid phase allowed to adsorb protein A, then adding endothelin labeled with HRP, and detecting the anti-endothelin monoclonal antibody bound to the solid phase. Selection and breeding of the anti-endothelin monoclonal antibody are usually achieved by addition of HAT (hypoxanthine, aminopterin and thymidine) and the use of a medium for animal cells containing 10 to 20% fetal calf serum, such as RPMI 1640. The antibody titer of the hybridoma culture supernatant can be measured in a similar manner as the above-mentioned method for measuring the titer of the anti-endothelin antibody in antiserum.

Separation and purification of the anti-endothelin monoclonal antibody are conducted in accordance with the method for separating and purifying immunoglobulin, similarly to the separation and purification of the polyclonal antibody described above.

The anti-endothelin monoclonal antibodies reactive to a partial region of endothelin can be prepared by the above-mentioned method using a peptide corresponding to this partial region as a hapten for immunization. Further, such anti-endothelin monoclonal antibodies can also be prepared by use of affinity chromatography employing a column combined with a peptide corresponding to the above partial region.

Screening for hybridoma cells producing the antiendothelin antibodies reactive to the partial region of endothelin and hybridoma cells producing the anti-endothelin monoclonal antibodies reactive to endothelin but not reactive to the partial region thereof can be accomplished, for example, by determining the activity between the antibody produced by the hybridoma cells and the peptide corresponding to the above partial region.

By using the anti-endothelin polyclonal antibody, the antiserum containing that antibody and the anti-endothelin monoclonal antibody thus obtained, determination and tissue staining of endothelin can be carried out according to immunoassays. Of the endothelin immunoassays are preferably used a competitive method and a sandwich method which will hereinafter described.

In the competitive method, the anti-endothelin antibody obtained by the present invention is competitively reacted with a test solution and labeled endothelin, followed by measurement of the ratio of labeled endothelin bound to the antibody to free labelled endothelin to determine the amount of endothelin contained in the test solution.

Labeling agents for endothelin or labeling agants for the antibody described hereinafter include radioisotopes, enzymes, fluorescent substances, luminous substances and the like. the radioisotopes include, for example, $^{125}I$, $^{131}I$; $^{3}H$ and $^{14}C$. The enzymes are preferable to be stable and high in specific activity, which include, for example, $\beta$- galactosidase, $\beta$-glucosidase, alkaline phosphatase, peroxidase and malate dehydrogenase. The fluorescent substances include fluorescamine, fluorescein isothiocyanate and the like. The luminous substances include luminol, luminol derivatives, luciferin and lucigenin. Further, a biotin-avidin system may be used in order to bind the labeling agents to the antibody or endothelin.

When the activity of the labeling agents described above is measured, it is necessary to separate labeled endothelin bound to the antibody from free labeled endothelin. This separation is hereinafter referred to as B/F separation for brevity.

When the enzyme is used as the labeling agent, there is advantageously used an active adsorbent such as an insolubilized antibody to the anti-endothelin antibody or insolubilized protein A, as a reagent for the separation. For example, an anti-IgG antibody (corresponding to the antibody to the anti-endothelin antibody) is used as the solid phase, and labeled endothelin is bound to the anti-IgG antibody of the solid phase through the foregoing antibody reactive thereto to measure the labeling agent on the solid phase. When the enzyme is used as the labeling agent, the activity of the enzyme on an insolubilized carrier is usually determined by fluorometric methods or fluorescent methods.

When the non-protein substance such as the radioisotope is used as the labeling agent, there are used for the B/F separation reagents other than the reagents described above, such as an antibody to the anti-endothelin which is not insolubilized, sodium sulfate, dextran charcoal powder and polyethylene glycol. In any methods, there is determined the activity of the labeling agent in the supernatant or in the precipitate.

The above insolubilization may be achieved by physical adsorption or chemical bonding usually used for insolubilizing or immobilizing proteins or enzymes. The carriers include polysaccharides such as agarose, dextran and cellulose, synthetic resins such as polystyrene, polyacrylamide and silicone, and glass.

In competitive methods, the anti-endothelin antibody, the test solution, labeled endothelin and the reagent for the B/F separation may be reacted in any order. Also, all or a part of them may be reacted at the same time. It is however preferable that at least labeled endothelin is added to the reaction system simultaneously with the reaction of the test solution and the anti-endothelin antibody, or after the reaction between the test solution and the anti-endothelin antibody was started.

The reagent for the B/F separation such as sodium sulfate, dextran charcoal powder or polyethylene glycol is mainly added to the reaction system at the final stage thereof.

In the sandwich method, the test solution is brought into contact with (or reacted with) an insolubilized anti- endothelin antibody (the first reaction), and further a labeled anti-endothelin antibody is reacted therewith (the second reaction), followed by measurement of the activity of the labeling agent on the insolubilized carrier to determine the amount of endothelin in the test solution. The first and second reactions may be conducted at the same time or at different times. The labeling agents and the insolubilizing methods conform to those described above.

As the anti-endothelin antibody used in the second reaction, there is preferably used the antibody different from the anti-endothelin antibody used in the first reaction in the site to which endothelin is bound. For example, if the antibody used in the first reaction has the binding capacity to the C-terminal portion of endothelin, in the second reaction is preferably used the anti-endothelin antibody which can be bound to a site other than the C-terminal portion, for example, to the N-terminal portion. On the other hand, if the antibody used in the first reaction has the binding capacity to the N-terminal portion of endothelin, in the second reaction is preferably used the anti-endothelin antibody which can be bound to a site other than the N-terminal portion, for example, to the C- terminal portion. Each of the antibodies used in the first and second reactions may be the polyclonal antibody or the monoclonal antibody. However, it is preferable that one is an anti-endothelin monoclonal antibody which reacts with endothelin, but does not react with the C-terminal portion of endothelin, and the other is an anti-endothelin polyclonal or monoclonal antibody which reacts with the C-terminal portion of endothelin.

In the immunoassay of endothelin I by the sandwich method, there are preferably used an anti-endothelin polyclonal antibody which reacts with the C-terminal peptide of endothelin I, namely Cys-His-Leu-Asp-Ile-Ile-Trp, and an endothelin monoclonal antibody which reacts with endothelin I, but does not react with the above C-terminal peptide of endothelin I.

In the immunoassay of endothelin II by the sandwich method, it is particularly preferred to use an anti-endothelin polyclonal antibody which reacts with the C-terminal peptide of endothelin II, for example, Val Asn Thr Pro Glu His Val Val Pro Tyr Gly Leu Gly Ser Pro Arg Ser or Val Asn Thr Pro Glu His Ile Val Pro Tyr Gly Leu Gly Ser Pro Ser Arg Ser, and an anti-endothelin monoclonal antibody which reacts with endothelin II, but does not react with the above C-terminal peptide of endothelin II.

Further, immuno-assays which use anti-endothelin antibodies of the present invention may be used for diagnosis or convalescent control of cardiac diseases or kidney diseases. Test samples may include body fluid such as blood plasma, serum, urine, cerebrospinal fluid, abdominal dropsy, thoracic fluid and amniotic fluid; phlegm and feces. These samples may be used as they are, or diluted with some buffers, or concentrated after being extracted. Solvents for dilution or extraction may include any suitable buffer or organic solvents. Preferred solvents include water, physiological saline, acetate buffer, acetone and chloroform-methanol, or those solutions including surface active agents.

Samples to by assayed in accordance with the present invention may be directly concentrated or concentrated after being applied to, e.g. an ion exchange carrier, a reverse phase chromatographic carrier or an anti-endothelin antibody bound carrier, and they are eluted with appropriate eluant. Concentration methods known to the art may be used, e.g. concentration under reduced pressure, normal pressure or in the nitrogen stream. As carriers for concentration, ODS cartridge for reverse-phase chromatography may most preferably used. The concentrate is dissolved in buffer for immuno assay and used as a sample for immuno assay.

Further, the anti-endothelin antibody obtained in the present invention can be used also for immune tissue staining. The methods therefor include, for example, the direct method using the labeled anti-endothelin antibody, and the indirect method using the anti-endothelin antibody and the labeled antibody to the anti-endothelin antibody.

Furthermore, of the anti-endothelin antibodies obtained by the present invention, the antibody which is able to neutralize the vasoconstrictor activity of endothelin can be employed as a specific antagonist to endothelin.

When amino acids, peptides, protective groups, active groups and so on are indicated by the abbreviations in this specification and drawings, the abbreviations adopted by IUPAC-IUB Commission on Biochemical Nomenclature or commonly used in the art are employed, for example, the following abbreviations are used. When the optical isomer is capable of existing with respect to the amino acids and so on, the L-form is represented unless otherwise specified. Trp : Tryptophan

Ile : Isoleucine

Leu : Leucine

Asp : Aspartic acid

Pro : Proline

Thr : Threonine

Gly : Glycine

His : Histidine

Cys : Cysteine

Arg : Arginine
Ser : Serine
Met : Methionine
Asn : Asparagine
Lys : Lysine
Glu : Glutamic acid
Gln : Glutamine
Val : Valine
Tyr : Tyrosine
Phe : Phenylalanine
Boc : t-Butoxycarbonyl
Tos : Tosyl
OBzl : Benzyl ester
MeBzl : 4-Methylbenzyl
Bzl : Benzyl
Cl-Z : 2-Chlorobenzyloxycarbonyl
Br-Z : 2-Bromobenzyloxycarbonyl
Acm : Acetoamidomethyl
TFA : Trifluoroacetic acid
TEA : Triethylamine
Pam : 4-(Oxymethyl)phenylacetoamidomethyl
pTs•PH : p-Toluenesulfonic acid
HONB : N-Hydroxy-5-norbornene-2,3-dicarboxyimide
ONB : N-Hydroxy-5-norbornene-2,3-dicarboxyimide ester
DMF : N, N′-Dimethylformamide
DCC : N,N′-dicyclohexylcarbodiimide
WSCD•HCl : 1-Ethyl-3-(3-dimethylaminopropyl)-carbodiimide hydrochloride

The present invention will hereinafter be described in detail with the following Examples. It is understood of course that these Examples are not intended to limit the scope of the invention.

Hybridoma cell AwETN40 used in the examples described later has been deposited in the Institute for Fermentation, Osaka, Japan (IFO) with accession number IFO 50168 since June 14, 1988. This hybridoma has also been deposited in Fermentation Research Institute, Agency of Industrial Science and Technology, Ministry of International Trade and Industry, Japen (FRI) with the accession number number FERM BP-1950 in accordance with the Budapest Treaty since July 12, 1988.

## Example 1

Preparation of Synthetic Peptides

### (1) Synthesis of Endothelin I

Endothelin I was synthesized by a conventional method, using 0.7 g (0.5m mole) of a commercially available Boc-Trp (CHO)-PAM resin (Applied Biosystems, U.S.A.) and a peptide synthesizer (Model 430A, Applied Biosystems, U.S.A.). The resin was treated with 50% trifluoroacetic acid in methylene chloride to deprotect a terminal amino group protected by a BOC group to a free amino group. To this free amino group, the following protected amino acids were condensed in order according to the amino acid sequence of endothelin I from the side of the C-terminus thereof in the presence of dicyclohexyl carbodiimide (DCC); Boc-Ile, Boc-Asp(OBzl), Boc-Leu, Boc-His(Tos), Boc-Cys(Acm), Boc-Tyr(Br-z), Boc-Val, Boc-Phe, Boc-Glu-(OBzl), Boc-Lys(Cl-Z), Boc-Met and Boc-Ser(Bzl)

With 1 ml of anithole and 1 ml of 1,2-ethanedithiol was swelled 800 mg of 1.8 g of the protected endothelin resin thus obtained, and then treated with 10 ml of hydrogen fluoride at 0°C for 60 minutes, followed by removal of excess hydrogen fluoride undler reduced pressure. After washing with 5 ml of ethyl acetate, the residue was extracted with 50% aqueous acetic acid and applied on a dextran gel column (Sephadex LH-2) (2 X 90 cm). Main fractions eluted with the above solvent were collected and then

7

lyophilized. Thus, 120 mg of white powder was obtained. 20 mg of the powder was dissolved in 20 ml of 80% aqueous acetic acid, and 15 mg of trifluoroacetate-mercury(II) was added thereto. After stirring at room temperature for 60 minutes, the resulting solution was diluted with 30 ml of the above solvent, and then hydrogen sulfide was passed therein. Thereafter, the precipitate generated thereby was filtered off, followed by lyophilization. The resulting product was dissolved in 400 ml of dilute acetic acid, adjusted to pH 8 with ammonium hydrogen carbonate, and then subjected to air- oxidation for 6 hours. Acetic acid was further added thereto to adjust it to pH 3, followed by lyophilization. The product thus obtained was applied on the Sephadex LH-20 column (2 X 90 cm) filled with 30% acetic acid to collect main fractions. The fractions were further fractionated by using HPLC (column: YMC, solvent: eluted by a linear gradient of 0.1% aqueous trifluoroacetic acid and acetonitril containing 0.1% trifluoroacetic acid). Thus, 2.7 mg of the desired product was obtained.

The synthesized endothelin I was eluted by HPLC at the same position as that of the natural extracted endothelin:

Assay conditions (1)

Column: Nucleosile 50 DS-H (Chemco) 4.6 mm$\phi$ X 250 mm)
Eluents: A solution (0.1% aqueous trifluoroacetic acid)
B solution (50% aqueous acetonitrile containing 0.1% trifluoroacetic acid)
A linear gradient elution from the eluent A to the eluent B for 20 minutes
Flow rate: 1.0 ml/minute

Assay conditions (2)

Column: DEAE-2SW (Toyo Soda Manufacturing Co.) (4.6mm$\phi$ X 250mm)
Eluents: A solution (10 mM Tris•HCl, pH 7.5)
B solution (A solution containing 1 M NaCl)
A linear gradient elution from the eluent A to the eluent B for 40 minutes
Flow rate: 1.0 ml/minute
Elution position: 21.5 minutes [assay conditions (1)]
21.3 minutes [assay conditions (2)]

(2) Synthesis of H-Cys-Leu-Asp-Ile-Ile-Trp-OH (C-Terminal portion of Endothelin I)

Boc-Ile-Trp-OBzl:>

In 100 ml of DMF was dissolved 84 g of H-Trp-OBzl-PTsOH, and neutralized with 25 ml of TEA. Thereafter, Boc-Ile-ONB prepared from 48o.9 g of Boc-Ile-OH•1/2H$_2$O, 40.12 g of HONB and 46.20 g of DCC was added thereto and stirred overnight. The solvent was removed therefrom by distillation under reduced pressure. The residue thus formed was dissolved by adding 400 ml of diethyl ether and 20 ml of ethyl acetate. The resulting solution was washed with 0.5 N HCl and 5% aqueous NaHCO$_3$, and then with water. Thereafter, the solution was dried with anhydrous MgSO$_4$. After the solvent was distilled off, petroleum ether was added to the residue and crystals were obtained by filtration.
The yield was 83.1 g (90.9%).
$[\alpha]_D^{13}$ = 19.7˚ (c -0.99 MeOH)
Elemental analysis: As C$_{29}$H$_{37}$N$_3$O$_5$
Calculated: C, 68.62; H, 7.35; N, 8.28
Found: C, 68.69; H, 7.35; N, 8.16
Boc-Ile-Ile-Trp-OBzl:
In 120 ml of dioxane was dissolved 53.3 g of Boc-Ile-Trp-OBzl, and 160 ml of 7.1 N HCl-dioxane was added thereto while being cooled with ice, followed by stirring for 90 minutes. After the solvent was removed therefrom by distillation undler reduced pressure, diethyl ether was added to the residue and then the precipitate was filtered. This precipitate was dissolved in a mixed solvent of 150 ml of acetonitrile and 250 ml of DMF and neutralized with 14.6 ml of TEA while being cooled with ice. Then, precipitated TEA·HCl was filtered off. To the filtrate, 45.33 g of Boc-Ile-ONB was added, and stirred overnight. After the solvent was removed by distillation, the residue was dissolved in 1 1 of CHCl₃, washed with 0.5 N HCl, 5% NaHCO₃ and water, and dried with anhydrous MgSO₄. After the solvent was distilled off undler reduced pressure, diethyl ether was added to the residue and the precipitate was filtered as crystals. Then, the precipitate was wlashed with acetonitrile and diethyl ether, and dried. The yield was 54.95 g (84.3%).

$[\alpha]_D^{18}$ - 12.0° (c = 0.98, CHCl₃) Elemental analysis: As $C_{35}H_{48}N_4O_6$

Calculated: C, 67.72; H, 7.79; N, 9.03

Found: C, 67.72; H, 7.91; N, 8.87

Boc-Asp-(OBzl)-Ile-Ile-Trp-OBzl:

In 250 ml of TFA containing 10% 1,2-ethanedithion was dissolved 49.7 g of Boc-Ile-Ile-Trp-OBzl while being cooled with ice and allowed to stand at room temperature for 15 minutes. The solvent was removed therefrom by distillation and 20 ml of 4 N HCl-dioxane was added to the residue, followed by stirring. Then, diethyl ether was added thereto and the precipitate was filtered. This precipitate was dissolved in 250 ml of DMF and neutralized with 11.1 ml of TEA while being cooled with ice. Then, precipitated TEA·HCl was filtered off. To the filtrate was added Boc-Asp-(OBzl)-ONB prepared from 31.0 g of Boc-Asp-(OBzl)-OH, 20.6 g of HONB and 23.8 g of DCC, and the resulting solution was stirred overnight. After the solvent was removed by distillation undler reduced pressure, 800 ml of ethyl acetate was added to the residue to dissolve it. The resulting solution was washed with 10% aqueous citric acid, 5% NaHCO₃ and water, and dried with anhydrous MgSO₄. After the solvent was distilled off undler reduced pressure, diethyl ether was added to the residue and the precipitate was filtered as crystals. Then, recrystallization from acetonitrile was conducted. The yield was 54.0 g (81.7%).

$[\alpha]_D^{18}$ - 13.7° (c = 1.04 CHCl₃)

Elemental analysis: As $C_{46}H_{59}N_5O_9$

Calculated: C, 66.89; H, 7.20; N, 8.48

Found: C, 66.69; H, 7.23; N, 8.31

Boc-Leu-Asp(OBzl)-Ile-Ile-Trp-OBzl:

In 210 ml of TFA containing 10% 1,2-ethanedithiol was dissolved 48.0 g of Boc-Asp(OBzl)-Ile-Ile-Trp-OBzl while being cooled with ice and allowed to stand at room temperature for 15 minutes. The solvent was removed therefrom by distillation and 14.5 ml of 4 N HCl-dioxane was added to the residue, followed by stirring. Then, diethyl ether was added thereto and the precipitate was filtered. This precipitate was dissolved in 300 ml of DMF and neutralized with 42 ml of TEA while being cooled with ice. Then, precipitated TEA·HCl was filtered off. To the filtrate was added 27.3 g of Boc-Leu-ONB and stirred overnight. After the solvent was removed by distillation under reduced pressure, 600 ml of CHCl₃ was added to the residue to dissolve it. The resulting solution was washed with 10% aqueous citric acid, 5% NaHCO₃ and water, and dried with anhydrous MgSO₄. After the solvent was distilled off under reduced plressure, petroleum ether was added to the residue and the precipitate was filtered. Then, recrystallization from hydrous acetonitrile was conducted.

The yield was 53.1 g (97.5%).

$[\alpha]_D^{18}$ - 20.6° (c = 1.02, CHCl₃)

Elemental analysis: As $C_{52}H_{70}N_6O_{10}·1/2H_2O$

Calculated: C, 65.87; H, 7.55; N, 8.86 Found: C, 66.08; H, 7.58; N, 8.78

Boc-His-Leu-Asp(OBzl)-Ile-Ile-Trp-OBzl:

In 220 ml of TFA containing 10% 1,2-ethanedithiol was dissolved 46.5 g of Boc-Leu-Asp(OBzl)-Ile-Ile-Trp-OBzl while being cooled with ice and allowed to stand at room temperature for 15 minutes. The solvent was removed therefrom by distillation under reduced pressure. Then, diethyl ether was added thereto and the precipitate was filtered. This precipitate was dissolved in 25 ml of DMF and 20 ml of TEA was added thereto, followed by stirring. Thereafter, 500 ml of diethyl ether was added thereto, and the precipitate was filtered as powder. Then, the powder was dissolved in 200 ml of DMF and Boc-His(Tos)-ONB prepared from 22.3 g of Boc-His(Tos)-OH, 11.7 g of HONB and 13.5 g of DCC was added thereto. The resulting solution was stirred overnight. After the solvent was distilled off, acetonitrile was added to the residue and the precipitated powder was filtered.

The yield was 45.9 g (86.2%).

$[\alpha]_D^{18}$ - 16.6° (c - 1.0, DMF)

Elemental analysis: As $C_{58}H_{77}N_9O_{11}·2.5H_2O$

Calculated: C, 62.13; H, 7.37; N, 11.24

Found: C, 62.25; H, 7.02; N, 11.03 Boc-Cys(MeBzl)-His-Leu-Asp(OBzl)-Ile-Ile-Trp-OBzl:

In 88 ml of TFA containing 10% 1,2-ethanedithiol was dissolved 17.2 g of Boc-His-Leu-Asp(OBzl)-Ile-Ile-Trp-OBzl while being cooled with ice and allowed to stand at room temperature for 15 minutes. The solvent was removed therefrom by distillation under reduced pressure. Then, ethyl ether was added to the residue and the precipitate was filtered as powder. This powder was dissolved in 25 ml of DMF and 25 ml of TEA was added thereto, followed by stirring. Thereafter, 500 ml of ethyl ether was added thereto, and the precipitate was filtered as powder. Then, the powder was dissolved in 40 ml of DMF and Boc-Cys(MeBzl)-ONB prepared from 5.72 g of Boc-Cys(MeBzl)-OH, 3.48 g of HONB and 4.00 g of DCC were added thereto. The resulting solution was stirred overnight. After the solvent was distilled off, acetonitrile was added to the residue and the precipitate was filtered as powder. The powder was further washed in hot acetonitrile, cooled to room temperature, and then filtered.

The yield was 15.0 g (72.9%).

$[\alpha]_D^{18}$ - 23.3˚ (c = 1.02, DMF)

Elemental analysis: As $C_{69}H_{90}N_{10}O_{12}S \cdot 3H_2O$

Calculated: C, 61.96; H, 7.23; N, 10.47; S, 2.40

Found: C, 62.24; H, 6.90; N, 10.49; S, 2.53

H-Cys-His-Leu-Asp-Ile-Ile-Trp-OH:

In 1 ml of TFA containing 10% 1,2-ethanedithiol was dissolved 100 mg of Boc-Cys(MeBzl)-His-Leu-Asp-(OBzl)-Ile-Ile-Trp-OBzl while being cooled with ice and allowed to stand at room temperature for 15 minutes. After the solvent was removed therefrom by distillation under reduced pressure, ethyl ether was added to the residue and the precipitate was filtered as powder. After dried, the powder was treated with 5 ml of HF in the presence of 0.5 ml of m-cresol at 0˚C for 1 hour. After dried, the powder was treated with 5 ml of HF in the presence of 0.5 ml of m-cresol at 0˚C for 1 hour. After HF was removed therefrom under reduced pressure, diethyl ether was added to the residue and the precipitate was filtered as powder. Then, the powder was washed with water. This powder was dissolved in 60% acetic acid and applied on a Sephadex LH-20 column (2.5 x 90 cm) filled with the same solvent. Fractions of 160 ml to 180 ml were collected and lyophilized.

The yield was 23 mg (32.8%)

Anal. for amino acids.

Asp 1.0(1), Cys 0.80(1),

Ile 2.13(2), Leu 0.99(1),

His 1.07(1) and Trp 0.67(1)

Average recovery 90.5%


(3) Synthesis of H-Arg-His-Leu-Asp-Ile-Ile-Trp-OH (Similar to C-Terminal Portion of Endothelin, with High Solubility)

In 5 ml of TFA containing 20% 1,2-ethanedithiol was dissolved 538 mg of Boc-His-Leu-Asp(OBzl)-Ile-Ile-Trp-OBzl while being cooled with ice and allowed to stand at room temperature for 15 minutes. After 95 mg of pTsOH·$H_2O$ was added thereto to dissolve it, the solvent was removed therefrom by distillation under reduced pressure. The residue was dissolved in 5 ml of DMF and neutralized by adding 0.75 ml of DMF containing 10% TEA thereto. Then, 60 mg of Boc-Arg($NO_2$)-OH, 50 mg of HONB and 50 mg of WSCD·HCl were added thereto, and the mixture was stirred overnight. After the solvent was distilled offer under reduced pressure, water was added to the residue and the precipitate was filtered. After dried, this precipitate was suspended in 10 ml of hot acetonitrile, cooled to room temperature, and then filtered.

The yield was 0.5 g (78.3%).

With HF was treated 102 mg of the resulting product in the presence of 0.2 ml of anisole and 0.2 ml of 1,2-ethanedithiol at 0˚C for 60 minutes, and then HF was removed by distillation under reduced pressure. After washing with diethyl ether, the residue was dissolved in 6 ml of 50% acetic acid and applied on a Sephadex G-25 (2.5 x 90 cm) column filled with the same solvent to develop it. Fractions of 100 ml to 137 ml were collected and lyophilized.

The yield was 20 mg (26.3%).

Anal. for amino acids:

Asp 0.98(1), Ile 1.95(2),

Leu 1.0(1), His 0.95(1),

Arg 0.98(1) and Trp 0.72(1)

Average recovery 90.5%

(4) Synthesis of Human Endothelin II (22 -38) (Human Endotheline C-Terminal Peptide)

Human endothelin II (22 - 38) was synthesized by a conventional method, using 0.685 g (0.5 m mole) of a commercially available Boc-Ser(Bzl)-PAM resin (Applied Biosystems, U.S.A.) and a peptide synthesizer (Model 430A, Applied Biosystems, U.S.A). The resin was treated with 50% trifluoroacetic acid in methylene chloride to deprotect a terminal amino group protected by a BOC group to a free amino group. To this free amino group, the following protected amino acides were condensed in order according to the amino acid sequence of human endothelin II (22 - 38) from the side of C-terminus thereof in the presence of dicyclohexyl carbodiimide (DCC);

Boc-ARg(Tos), Boc-Pro, Boc-Ser(Bzl), Boc-Gly, Boc-Leu, Boc-Tyr(Br-Z), Boc-Val, Boc-His(Tos), Boc-Glu(OBzl), Boc-Thr(Bzl), and Boc-Asn

With 1 ml of m-cresol was swelled 881 mg of 1.783 g of the protected peptide resin thus obtained, and then, treated with 10 ml of hydrogen fluoride at 0°C for 60 minutes, followed by removal of excess hydrogen fluoride under reduced pressure. After washing with 10 ml of diethyl ether the residue was filtered on a glass filter and 10 ml of 50% acetic acid was added thereto to dissolve the peptides. The resin was further washed twice with 5 ml of 50% acetic acid. The filtrate and washings were combined and then concentrated to 1/10 of the original volume at room temperature under reduced pressure, followed by addition of 20 ml of water to dilute it. Thereafter, the diluted solution was passed through an Amerlite IRA-400 resin (acetic acid type) column (1 X 3 cm) and then lyophilized. The resulting product was applied on a Sephadex LH-20 column (2 X 90 cm) filled with 50% acetic acid and eluted with the same solvent. Main fractions were collected and then lyophilized. Thus, 322 mg of white powder was obtained.
Anal. for amino acids:
Asp 1.00(1), Thr 0.94(1),
Ser 1.72(2), Glu 1.13(1),
Gly 2.0(2), Val 2.51(3),
Leu 1.03(1), Tyr 0.67(1),
His 0.95(1), Arg 0.95(1) and
Pro 3.00(3)
Average recovery 82.9%


(5) Synthesis of Human Endotheline II (1 - 38)

Human endothelin II (1 - 38) was synthesized by a conventional method, using 0.685 g (0.5 m mole) of a commercially available Boc-Ser(Bzl)-PAM resin (Applied Biosystems, U.S.A.) and a peptide synthesizer (Model 430A, Applied Biosystems, U.S.A.). The resin was treated with 50% trifluoroacetic acid in methylene chloride to deprotect a terminal amino group protected by a BOC group to a free amino group. To this free amino group, the following protected amino acids were condensed in order according to the amino acid sequence of human endothelin II (1 - 38) from the side of the C-terminus thereof in the presence of dicyclohexyl carbodiimide (DCC);

Boc-Arg(Tos), Boc-Pro, Boc-Ser(Bzl), Boc-Gly, Boc-Leu, Boc-Tyr(Br-Z), Boc-Val, Boc-His(Tos), Boc-Glu(OBzl), Boc-Thr(Bzl), Boc-Asn, Boc-Trp(CHO), Boc-Ile, Boc-Asp(OBzl), Boc-Cys(MeBzl), Boc-Lys(Cl-Z) and Boc-Met

Each condensation reaction was repeated until it was confirmed by the ninhydrin test that the reaction had been approximately completed.

With 0.4 ml of m-cresol and 0.6 ml of 1,2-ethanedithiol was swelled 500 mg of 2.50 g of the protected peptide resin thus obtained, and then treated with 10 ml of hydrogen fluoride at 0°C for 60 minutes, followed by removal of excess hydrogen fluoride under reduced pressure. After washing with 10 ml of diethyl ether, the residue was extracted with 6 ml of trifluoroacetic acid. The resin was filtered and then washed with 2 ml of trifluoroacetic acid. The filtrate and washings were combined, and poured in 300 ml of water while cooling with ice. Then, aqueous ammonia was added thereto to adjust it to pH 8 and it was mildly stirred overnight. The white suspended material was centrifuged at a rotation speed of 3,500 rpm and the supernatant was applied on a Diaion HF-20 column (1 x 2.5 cm). After the column was washed with 200 ml of water, elution with 100 ml of 80% aqueous methanol and concentration were conducted. The resulting product was applied on a Sephadex LH-20 column (2 X 90 cm) filled with 50% acetic acid and main fractions were collected. The fractions were further fractionated by using HPLC (column: YMC-ODS, solvent: eluted by a linear gradient of 0.1% aqueous trifluoroacetic acid and acetonitrile containing 0.1% trifluoroacetic acid, or 27% aqueous acetonitrile containing 0.1% trifluoroacetic acid). Thus, 2 mg of the desired product was obtained.

Anal. for amino acids:

Asp 2.81(3), Thr 0.95(1),

Ser 4.03(5), Glu 1.94(2),

Gly 2(2), Val 3.73(4),

Met 0.86(1), Ile 1.12(2),

Leu 2.96(3), Tyr 1.90(2),

Phe 0.95(1), Lys 1.01(1),

His 1.89(2), Trp 0.95(1)

Arg 0.92(1) and Pro 3.04(3)

Average recovery 60.2%

(6) Synthesis of Porcine Endothelin II (1 - 39)

Porcine endothelin II (1 - 39) was synthesized by a conventional method, using 0.685 g (0.5 m mole) of a commercially available Boc-Ser(Bzl)-PAM resin (Applied Biosystems, U.S.A) and a peptide synthesizer (Model 430A, Applied Biosystems, U.S.A.). The resin was treated with 50% trifluoroacetic acid in methylene chloride to deprotect a terminal amino group protected by a BOC group to a free amino group. To this free amino group, the following protected amino acids were condensed in order according to the amino acid sequence of porcine endothelin II from the side of the C-terminus thereof in the presence of dicyclohexyl carbodiimide (DCC);

Boc-Arg(Tos), Boc-Pro, Boc-Ser(Bzl), Boc-Gly, Boc-Leu, Boc-Tyr(Br-Z), Boc-Val, Boc-His(Tos), Boc-Glu-(OBzl), Boc-Thr(Bzl), Boc-Asn, Boc-Trp(CHO), Boc-Ile, Boc-Asp(OBzl), Boc-Cys(MeBzl), Boc-Lys(Cl-Z) and Boc-Met

Each condensation reaction was repeated until it was confirmed by the ninhydrin test that the reaction had been approximately completed.

With 0.4 ml of m-cresol and 6 ml of 1,2-ethanedithiol was swelled 500 mg of 2.32 g of the protected peptide resin thus obtained, and then treated with 10 ml of hydrogen fluoride at 0°C for 60 minutes, followed by removal of excess hydrogen fluoride under reduced pressure. After washing with 10 ml of diethyl ether, the residue was extracted with 6 ml of trifluoroacetic acid. The resin was filtered and then washed with 2 ml of trifluoroacetic acid. The filtrate and washings were combined, and poured in 300 ml of water while cooling with ice. Then, aqueous ammonia was added thereto to adjust it to pH 8 and it was mildly stirred overnight. The white suspended material was centrifuged at a rotation speed of 3,500 rpm and the supernatant was applied on a Diaion HP-20 column (1 X 2.5 cm). After the column was washed with 200 ml of water, elution with 100 ml of 80% aqueous methanol and concentration were conducted. The resulting product was applied on a Sephadex LH-20 columns (2 X 90 cm) filled with 50% acetic acid and main fractions were collected. The fractions were further fractionated by using HPLC (column: YMC-ODS, solvent: eluted by a linear gradient of 0.1% aqueous trifluoroacetic acid and acetontrile containing 0.1% trifluoroacetic acid, or 27% aqueous acetontrile containing 0.1% trifluoroacetic acid). Thus, 3 mg of the desired product was obtained.

Anal. for amino acids:

Asp 2.73(3), Thr 0.91(1),

Ser 5.03(6), Glu 2.01(2),

Gly 2(2), Val 2.1(3),

Met 0.91(1), Ile 2.03(3),

Leu 2.92(3), Tyr 1.88(2),

Phe 0.91(1), Lys 1.01(1),

His 1.88(2), Trp 1.02(1)

Arg 0.97(1) and Pro 2.80(3)

Average recovery 59.2%

Example 2

Preparation of Immunogens

(1) Immunogen (I)

Endothelin I obtained in Example 1 was bound to bovine thyroglubulin (hereinafter referred to as TG for brevity) by using glutaraldehyde. That is to say, 4.5 mg of the above polypeptide and 13.5 mg of TG were dissolved in 4.5 ml of 0.2 M phosphate buffer (pH 7.0) and then glutaraldehyde was added thereto so as to be finally adjusted to a concentration of 0.2%. The reaction was conducted at room temperature for 3 hours. After the reaction was completed, the resulting product was dialyzed against a physiological saline for 2 days.

(2) Immunogen (II)

The condensation product of the polypeptide Cys His Leu Asp Ile Ile Trp obtained in Example 1 and bovine serum albumin (hereinafter referred to as BSA for brevity) was prepared according to the maleimide cross-linking method described below to provide immunogen (II).

Namely, 20 mg of BSA was dissolved in 1.4 ml of 0.1 M phosphate buffer (pH 7.0), and 100 $\mu$l of DMF solution containing 2.6 mg of N-($\gamma$-maleimidebutyryloxy) succinimide (hereinafter referred to as GMBS for brevity) was mixed therewith. The reaction was conducted at room temperature for 40 minutes. After the reaction was completed, the resulting product was fractionated on a Sephadex G-25 column previously equilibrated with 0.1 M phosphate buffer (pH 6.5) containing 2.5 mM EDTA. Then, 1.2 ml of the eluted fractions containing 5.4 mg of BSA having maleimide groups introduced therein was reacted with 1.8 mg of the polypeptide Cys His Leu Asp Ile Ile Trp dissolved or dispersed in 1.2 ml of 90% aqueous dimethyl sulfoxide, at 4°C for 3 days. After the reaction was completed, the product was dialyzed against a physiological saline at 4°C for 2 days.

(3) Immunogen (III)

Human endotheline II C-terminal polypeptide obtained in (6) of Example 1, Val Asn Thr Pro Glu His Val Val Pro Tyr Gly Leu Gly Ser Pro Arg Ser, and TG were condensed by the maleimide cross-linking method described below to provide immonogen (III).

That is to say, 1.63 $\mu$moles of the above polypeptide was dissolved in 350 $\mu$l of 0.1 M phosphate buffer (pH 7.0), and 100 $\mu$l of a DMF solution containing 9.1 mg (33 $\mu$moles) of GMBS was mixed therewith. The reaction was conducted at room temperature for 30 minutes. After the reaction was completed, the resulting product was fractionated on a Sephadex G-15 column. Thus, 1.09 $\mu$moles of the polypeptide having maleimide groups introduced therein was obtained.

On the other hand, 20 mg (40 $\mu$moles) of TG was dissolved in 1.4 ml of 0.02 M phosphate buffer (pH 6.8) containing 0.15 M NaCl, and 100 ml of a DMF solution containing 2.5 mg (8.0 $\mu$moles) of N-succinimidyl-3-(2-pyridyldithio) propionate (hereinafter referred to as SPDP for brevity) was mixed therewith. The reaction was conducted at room temperature for 40 minutes. After the reaction was completed, 0.5 ml of 0.1 M acetate buffer (pH 4.5) containing 12.4 mg (80 $\mu$moles) of dithiothreitol was added thereto and the reaction was further performed at room temperature for 20 minutes. Then, the resulting product was fractionated on a Sephadex G-25 column. Thus, 12 mg (24 nmoles) of TG having SH groups introduced therein was obtained.

Subsequently, 1.09 $\mu$moles fo the maleimide group-introduced endothelin II C-terminal polypeptide and 12 nmoles of the SH group-introduced TG were mixed with each other and reacted at 4°C for 2 days. Thereafter, the resulting product was dialyzed against a physiological saline at 4°C for 2 days.

Example 3

Immuninization

Three kinds of emulsions were prepared by adding 550 $\mu$l of Freund's complete adjuvant to each of 450 $\mu$l physiological salines containing 400 $\mu$g of immunogen (I), 600 $\mu$g of immunogen (II) or 450 $\mu$g of immonogen (III) obtained in Example 2, respectively and mixing them thoroughly. Each of the emulsions was subcutaneously inoculated on each of the rabbits at about 20 spots. After 6 weeks from the inoculation, each of emulsions similarly prepared by using Freund's incomplete adjuvant was subcutaneously inoculated on each of the rabbits. This operation was repeated 3 times for immunogen (I), and 4 times for immunogen (II) and immunogen (III), at monthly intervals. After 7 days from supplemental immunization, bloods were partially collected from the rabbits and antisera were obtianed using methods known in the art.

Similarly, 70 μg/mouse of immunogen (I) and Freund's complete adjuvant were subcutaneously inoculated on female mice BALB/C 6 to 8 weeks old. Thereafter, supplemental immunization was carried out 2 to 3 times at 3-week intervals. After 7 days from the supplemental immunization, blood was collected from each of the mice and antisera were obtained.

Example 4

Preparation of Enzyme-Labeled Antigens

(1) Preparation of Peroxidase-Labeled Endothelin I

In 450 μl of 0.1 M phosphate buffer (pH 7.0) was dissolved 210 nmoles of endothelin I, and 50 μl of a DMF solution containing 295 μg (1.05 μmoles) of GMBS was mixed therewith. The reaction was conducted at room temperature for 30 minutes. After the reaction was completed, the resulting product was fractionated on a Sephadex G-15 column. Thus, 100 nmoles of the polypeptide having maleimide groups introduced therein was obtained.

On the other hand, 10 mg (250 nmoles) of horseradish peroxidase was dissolved in 1.4 ml of 0.02 M phosphate buffer (pH 6.8) containing 0.15 M NaCl, and 100 μl of a DMF solution containing 1.17 mg (3.75 μmoles) of SPDP was mixed therewith. The reaction was conducted at room temperature for 40 minutes. After the reaction was completed, 0.5 ml of 0.1 M acetate buffer (pH 4.5) containing 12.4 mg (80 μmoles) of dithiothreitol was added thereto and the reaction was further conducted at room temperature for 20 minutes. Then, the resulting product was fractionated on a Sephadex G-25 column. Thus, 6 mg (150 nmoles) of peroxidase having SH groups introduced therein was obtained.

Then, 50 nmoles of maleimide group-introduced endothelin I and 10 nmoles of SH group-introduced peroxidase were mixed and reacted with each other at 4°C for 16 hours. After the reaction was completed, the resulting product was fractionated on a Ultrogel AcA44 column (LKB-Pharmacia) to obtain peroxidase-labeled endotheline I.

(2) Preparation of Peroxidase-Labeled Human Endothelin II C-Terminal Peptide

In 450 μl of 0.1 M phosphate buffer (pH 7.0) was dissolved 690 μg (380 nmoles) of the human endothelin II C-terminal peptide, and 50 μl of DMF solution containing 295 μg (1.05 μ moles) of GMBS was mixed therewith. The reaction was conducted at room temperature for 30 minutes. After the reaction was completed, the resulting product was fractionated on a Sephadex G-15 column. Thus, 230 nmoles of the polypeptide having maleimide groups introduced therein.

Then, this peptide and 40 nmoles of SH group-introduced peroxidase prepared according to the method described in the preceding (1) of this example were mixed and reacted with each other at 4°C for 16 hours. After the reaction was completed, the resulting product was fractionated on a Ultrogel AcA44 column (LKB-Pharmacia) to obtain peroxidase-labeled endothelin II C-terminal peptide.

Example 5

Assay of Antibody Titer

The titers of the rabbit antiserum antibnodies to immunogen I were determined by the method described below. Namely, 100 μl portions of 0.1 M sodium carbonate buffer (pH 9.6) containing 20 μg/ml of anti-rabbit IgG antibody (IgG fraction, Kappel) were separately poured into each well of a 96-well microtiter plate, and allowed to stand at 4°C for 24 hours. After the plate was washed with a phosphate buffer physiological saline (hereinafter referred to as PBS for brevity), 300 μl portions of PBS containing 1% BSA were separately poured into each well to block excess binding sites of the wells, and treated at a temperature of at least 4°C for 24 hours.

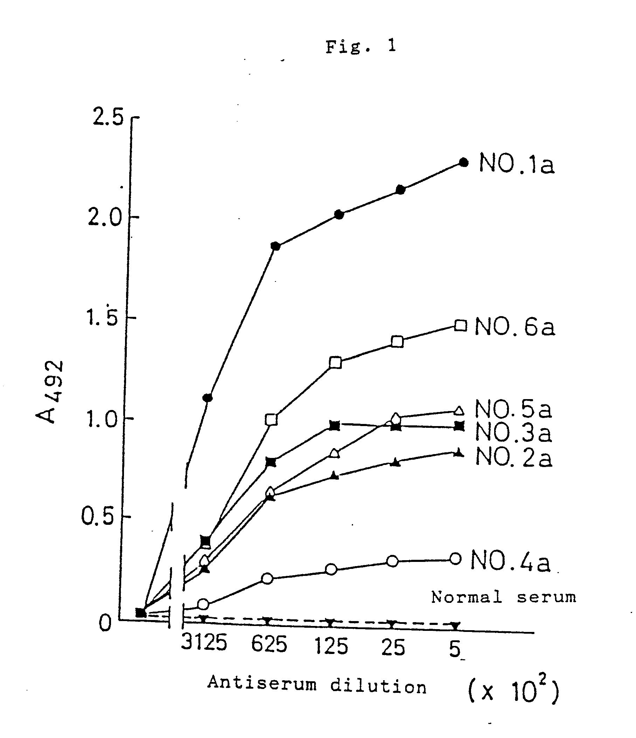

To the plate prepared as described above were added 50 μl of each rabbit antiserum (6 rabbits, Nos. 1a to 6a) diluted with buffer A (0.02 M phosphate buffer, 1% BSA, pH 7.0) and 50 μl of the above peroxidase-labeled endothelin I (diluted 100 times with buffer A), and the reaction was conducted at 4°C for 16 hours. After the reaction was completed, the plates were washed with PBS. Then, in order to assay the enzyme activity on the solid phase thus prepared, 100 μl portions of 0.1 M citric acid buffer (pH 5.5) containing 0.2% o-phenylenediamine and 0.02% hydrogen peroxide were separately poured into each well, and the reaction was conducted at room temperature for 10 minutes. After 100 μl of 4 N sulfuric acid was added thereto to terminate the reaction, the absorption at 492 nm was measured by a plate reader (MTP-32, Corona) to assay the antibody activity. The results are shown in Fig. 1.

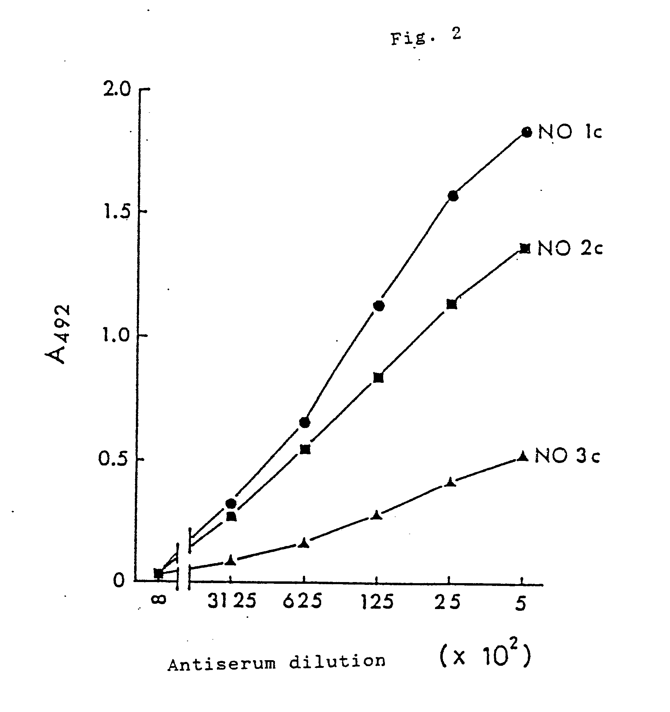

Similarly, the titer of the antibody in each of the rabbit antisera (3 rabbits, Nos. 1c to 3c) to immunogen II was assayed. The results are shown in Fig. 2.

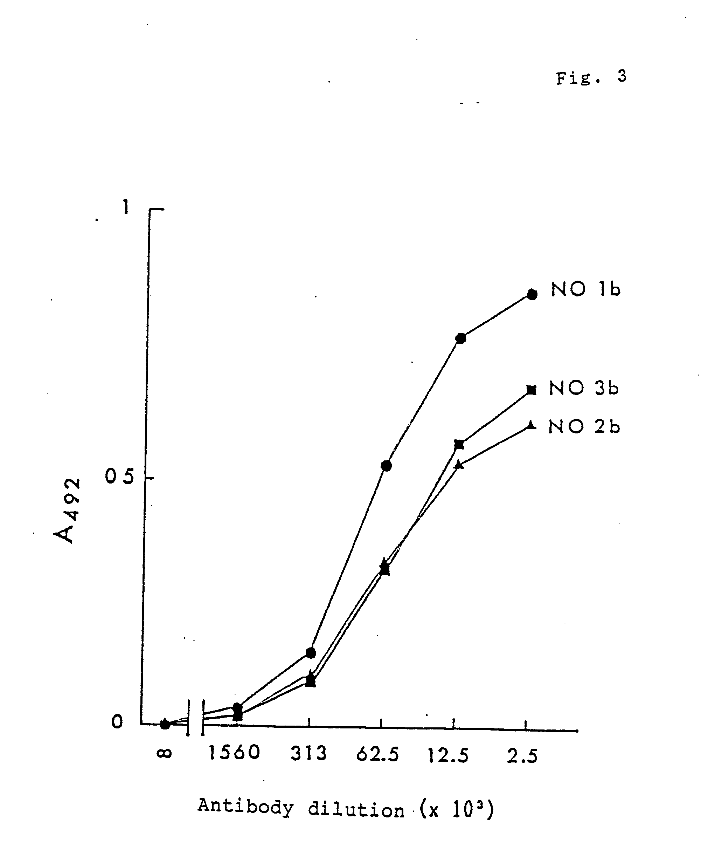

Futher, the titer of the antibody in each rabbit antiserum (3 rabbits, Nos. 1b to 3b) to immunogen III was similarly assayed, using the peroxidase-labeled human endothelin II C-terminal peptide described in (2) of Example 4 as a conjugate. The results are shown in Fig. 3.

Furthermore, the titers of the mouse antiserum antibodies to immunogen I were determined by the method described below. Namely, 100 μl portions of 0.1 M sodium carbonate buffer (pH 9.6) containing 20 μg/ml of anti-mouse IgG antibody (IgG fraction, H-chain and L-chain specificities, Kappel) were separately poured into each well of a 96-well microtiter plate, and allowed to stand at 4°C for 24 hours. After the plate was washed with PBS, 300 μl portions of PBS containing 1% BSA were separately poured into each well to block excess binding sites fo the wells, and treated at a temperature of at least 4°C for 24 hours.

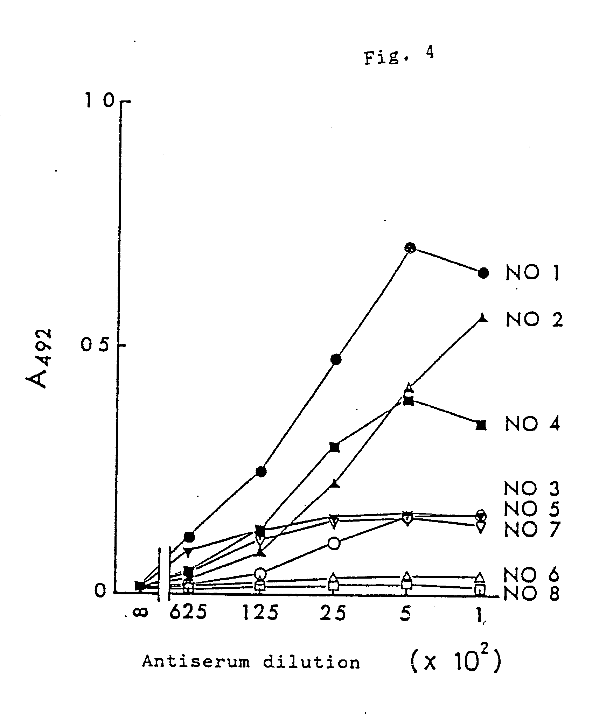

To the plate prepared as described above were added 50 μl of each mouse antiserum (8 mice, Nos. 1a to 8a) diluted with buffer A and 50 μl of the above peroxidase-labeled endothelin I (diluted 100 times with buffer A), and the reaction was conducted at 4°C for 16 hours. After the reaction was completed, the plates were washed with PBS. Then, the enzyme activity on the solid phase thus prepared was determined by the method described above. The results are shown in Fig. 4.

Further, the anti-endothelin antibody producing hydridoma cells were selected by measuring the titers of the antibodies in hybridoma culture supernatants according to the method similar to that described above.

Example 6

Preparation of Affinity Solid Phases

(1) Affinity solid phase (I)

The condensation product of the polypeptide Cys His Leu Asp Ile Ile Trp and human serum albumin (hereinafter referred to as HSA for brevity) was bound to CNBr-activated Sepharose 4B (Pharmacia) to provide affinity solid phase (I).

That is to say, 20 mg of HSA was reacted with 2.1 mg of GMBS at room temperature for 60 minutes and the reaction product was fractionated on a Sephadex G-25 column, by the method described in Example 2. Then, 1 ml of the eluted fractions containing 5 mg of HSA having maleimide groups introduced therein was reacted with the polypeptide Cys His Leu Asp Ile Ile Trp dissolved or dispersed in 1 ml of an aqueous solution containing 90% dimethyl sulfoxide at 4°C for 3 days. After the reaction was completed, the resulting product was dialyzed against 0.1 M sodium hydrogencarbonate solution containing 0.5 M NaCl and then reacted with 1 g of CNBr-activated Sepharose 4B at room temperature for 3 hours. After treated with 0.1 M tris-hydrochloric acid buffer (pH 8) to remove the unreacted active groups therefrom, the affinity solid phase thus prepared was dispersed in PBS containing 1% BSA and stored at 4°C.

(2) Affinity Solid Phase (II)

The polypeptide Arg His Leu Asp Ile Ile Trp was directly bound to CNBr-activated Sepharose 4B to provide affinity solid phase (II).

Namely, 1.5 mg of the polypeptide Arg His Leu Asp Ile Ile Trp was dissolved in 10 ml of 0.1 M sodium hydrogencarbonate solution containing 0.5 M NaCl and then reacted with 1 g of CNBr-activated Sepharose 4B at room temperature for 3 hours. After treated with 0.1 M tris-hydrochloric acid buffer (pH 8) to remove the unreacted active groups therefrom, the affinity solid phase thus prepared was dispersed in PBS and stored at 4°C.

### (3) Affinity Solid Phase (III)

The human endothelin II C-terminal peptide was directly bound to CNBr-activated Sepharose 4B to provide affinity solid phase (III).

Namely, 1.5 mg of the above peptide was dissolved in 10 ml of 0.1 M sodium hydrogencarbonate solution containing 0.5 M NaCl and then reacted with 1 g of CNBr-activated Sepharose 4B at room temperature for 3 hours. After treatment with 0.1 M tris-hydrochloric acid buffer (pH 8) to remove the unreacted active groups therefrom, the affinity solid phase thus prepared was dispersed in PBS and stored at 4°C.

## Example 7

Purification of Anti-Endotheline Polyclonal Antibodies

### (1) Purification by Anion Exchange Chromatography

The rabbit anti-endothelin I antiserum was fractionated by DEAE-cellulose column chromatography after the salting out thereof, whereby the anti-endotheline I antibody was purified to IgG fractions.

That is to say, 10 ml of PBS was added to 10 ml of the rabbit anti-endothelin I antiserum No.1a and 16.5 ml of a saturated ammonium sulfate solution was further added slowly thereto while being stirred (to a final concentration of 45%). After allowed to stand for 30 minutes, the resulting mixture was centrifuged at 12,000 X g for 20 minutes and the precipitate formed thereby was dissolved in 10 ml of PBS. Thereafter, a saturated ammonium sulfate solution was similarly added thereto so that a final saturated concentration of 30% was reached, followed by centrifugation. The precipitate formed thereby was dissolved in 10 ml of 0.01 M borate buffer saline (pH 8) (hereinafter referred to as BBS for brevity) containing 0.15 M NaCl. Then, the solution was dialyzed against 0.01 M phosphate buffer (pH 8) (hereinafter referred to as buffer B) containing 0.01 M NaCl at 4°C for 2 days.

Subsequently, the antibody fraction obtained by the above salting out and dialysis were added to a DEAE-cellulose column (DE-52, Wattman, 20 mmØX 100 mm) previously equilibrated with buffer B. After the column was washed with buffer B, the antibody was eluted by using a continous ionic strength gradient from buffer B to buffer C (0.01 M phosphate buffer containing 0.35 M NaCl, pH 8). The titer of the antibody in the eluted fractions was determined according to the method described in Example 5.

The detection of rabbit immunoglobulins was carried out by the method similar to that described in Example 5, using the antibody specific to each immunoglobulin as a solid phase. Namely, the anti-rabbit IgGFc component or anti-rabbit IgM (goat antibody, IgG fraction, Kappel) was fixed to a microtiter plate according to the method described in Example 5. Then, 50 $\mu$l of each fraction of DEAE-cellulose-eluted anti-endothelin antibodies diluted $10^2$ to $10^7$ times with buffer A and 50 $\mu$l of peroxidase-labeled endothelin I diluted 100 times with buffer A were added thereto, and the reaction was conducted at 4°C for 16 hours. After the reaction was completed, the resulting product was washed and then the enzyme activity on each solid phase was assayed. As a result, when the anti-rabbit IgGFc component was used as a solid phase, the most active reaction was observed. Also when anti-rabbit IgM was used as a solid phase, the specific reaction was observed.

### (2) Purification by Affinity Solid Phase (i)

The antibodies were partially purified from 8 ml portions of rabbit antisera Nos. 1c and 1d against immunogen (II) by the ammonium sulfate salting-out method described in (1) of Example 7. After dialyzed against BBS, the antibody fractions were applied on columns (10 mm ∅X 40 mm) filled with the affinity solid phase (I) described in (1) of Example 6. After thorough washing with BBS, specific antibodies were eluted with 0.1 M acetate buffer (pH 4.5) containing 0.1 M NaCl, and further with 0.05 M glycine-hydrochloric acid buffer (pH 2.0) containing 0.1 M NaCl. The titers of the antibodies in the eluted fractions were assayed by the EIA method described in Example 5. As a result, a high antibody titer was observed only in the fraction eluted at pH 2. From that fraction, 18 mg of a specific antibody was obtained.

Similarly, the antibodies were purified from 6 ml portions of rabbit antisera Nos. 1b and 1c against immunogen (I) by the ammonium sulfate salting-out method. After dialysis against BBS, the antibody fractions were applied on columns (10 mm ∅ X 40 mm) filled with the affinity solid phase (II) described in (2) of Example 6. Then, specific antibody fractions were eluted according to the method described above. Consequently, 5.4 mg of a specific antibody was obtained from the fraction eluted at pH 2.

(3) Purification by Affinity Solid Phase (ii)

The antibodies were partially purified from 14 ml portions of rabbit antisera Nos. 1b and 1c against immunogen (III) by the ammonium sulfate salting-out method described in (1) of Example 7. After dialysis against BBS, the antibody fractions were applied on columns (10 mm ∅ X 40 mm) filled with the affinity solid phase (III) described in (3) of Example 6. After thorough washing with BBS, specific antibodies were eluted with 0.1 M acetate buffer (pH 4.5) containing 0.1 M NaCl, and further with 0.05 M glycine-hydrochloric acid buffer (pH 2.0) containing 0.1 M NaCl. The titers of the antibodies in the eluted fractions were assayed by the EIA method described in Example 5. As a result, a high antibody titer was observed only in the fraction eluted at pH 2. From that fraction, 12 mg of a specific antibody was obtained.

Example 8

Cell Fusion

The final immunization was performed to the mice Nos. 1 and 4 which showed relatively high antibody titers, by inoculating in their veins 0.25 ml of a physiological saline in which 240 μg of immunogen (I) was dissolved. The spleens were taken out of the mice Nos. 1 and 4 after 3 days from the final immunization, pressed by a stainless mesh, filtered and floated in Eagle's minimum essential mediums (MEM), whereby spleen cell-floating solutions were obtained. As the cells for cell fusion, myeloma cells derived from BALB/C mouse, P3-X 63.Ag8.U1 (P3U1), were used [Current Topics in Microbiology and Immunology, 81, 1 (1978)]. The cell fusion was carried out according to the original method [Nature, 256, 495 (1975)]. Namely, the spleen cells and P3U1 were washed 3 times by serum-free MEM, respectively, and mixed in a ratio of the spleen cells to P3U1 5 : 1. The mixture was centrifuged at 800 rpm for 15 minutes to precipitate the cells. After the supernatant was removed, 0.3 ml of 45% polyethylene glycol (PEG) 6000 (Kochlight) was added to the precipitate lightly loosened. Then, the mixture was allowed to stand in a hot water bath at 37° C for 7 minutes, whereby the fusion was performed. After the fusion was completed, MEM was added to the cells at a rate of 2 ml per minute. After the total amount of added MEM reached 12 ml, the supernatant was removed by centrifugation at 600 rpm for 15 minutes. This cell precipitate was floated in RPMI 1640 medium (RPMI 1640- 10 FCS) containing 10% fetal calf serum so that P3U1 was contained in an amount of $2 \times 10^6$ cells per ml. This was seeded in 120 wells (24-well multi-dish, Linbro) in an amount of 1 ml per well. After seeding, the cells were cultured in a 5% carbon dioxide incubator at 37° C. After 24 hours of culture, 1 ml portions of RPMI 1640-10 FCS medium (HAT medium) containing HAT ($1 \times 10^{-4}$ M hypoxanthine, $4 \times 10^{-7}$ M aminopterin and $1.6 \times 10^{-3}$ M thymidine) were added to each well to initiate HAT selective culture. HAT selective culture was continued by discarding 1 ml of old liquor and then supplying 1 ml of HAT medium 3, 6 and 9 days after the initiation of the culture. The propagation of hybridoma cells were observed 9 to 14 days after the cell fusion had been completed. Wlhen the culture solution turned yellow (about $1 \times 10^6$ cells/ml), the supernatants were collected and the antibody titers thereof were determined by the EIA method described in Example 5. Thus, the supernatants of all 120 wells in which the propagation of hybridoma cells was observed were investigated. As a consequence, the high antibody activity was observed in 3 wells (Nos. 1-25, 1-27 and 1-86) when the spleen cells fo mouse No. 1 were used for the cell fusion, and in 2 wells (Nos. 4-61 and 4-80) when the spleen cells of mouse No. 4

17

were used for the cell fusion.

Example 9

Cloning

Of the wells which showed the positive antibody activity, the hybridoma cells fo each of 3 wells (Nos. 1-25, 1-86 and 4-80) were cloned by the limiting dilution method. That is to say, the hybridoma cells were floated in RPMI 1640-20 FCS so as to be contained in an amount of 1.5 cells/ml and 0.2 ml portions thereof were separately poured into each well of a 96-well microtiter plate (Nunk). Upon pouring, the thymocytes of BALB/C mouse were added thereto as feeder cells so as to be contained in an amount of $5 \times 10^5$ cells per well. After about one week, the propagation of the cells was observed. The titers of the antibodies in the supernatants were examined by the EIA method described in Example 5. As a result, the antibody activity was observed in 16 clones of 28 clones for No. 1-25, 13 clones of 107 clones for No. 1-86 and 5 clones of 131 clones for No. 4-80.

Giving attention to clone AwETN40 obtained from No. 4-80 of these clones and monoclonal antibody AwETN40a produced thereby, the following experiments were carried out.

Example 10

Preparation of Large Amount of Monoclonal Antibody

Into a mouse to which 0.5 ml of mineral oil had been intraperitoneally administered or an untreated mouse (BALB/C), $1 \times 10^6$ to $3 \times 10^6$ cells/mouse of hybridoma AwETN40 was intraperitoneally injected. After 10 to 30 days, the antibody-containing ascites was collected.

Example 11

Purification of Monoclonal Antibody

Monoclonal antibody AwETN40a was purified by salting out the ascites obtained in Example 10 according to the method described in Example 7 and then fractionating it by DEAE-cellulose column chromatography.

Namely, 7 ml of PBS was added to 7 ml of the ascites, and 11.5 ml of saturated ammonium sulfate was further added slowly thereto (to a final concentration of 45%). The precipitate was dissolved in BBS, and the resulting solution was dialyzed against 0.01 M phosphate buffer (pH 8) containing 0.01 M NaCl. Then, the fractions were applied on a DEAE-cellulose column (20 mm $\emptyset$ X 100 mm). The antibody was eluted by a concentration gradient of 0.01 M to 0.35 M NaCl, whereby 90 mg of monoclonal antibody AwETN40a was obtained from 7 ml of the ascites as a purified sample.

Example 12

Determination of Class and Subclass of Monoclonal Antibody

Into each well of a 96-well microtiter plate, 100 μl portions of 0.1 M carbonate buffer (pH 9.6) containing 2 μg/ml of the purified monoclonal antibody obtained in Example 11 were separately poured and allowed to stand at 4° C for 24 hours. After the excess binding sites of the wells were blocked with BSA, the class and subclass of the monoclonal antibody were examined by the enzyme-linked immunosorbent assay (ELISA) using an isotype typing kit (Mouse TyperTM Sub-Isotyping Kit, Bio RAD). As a result, it was found that AwETN40a belonged to IgGl, x class.

18

Example 13

Examination of Neutralization Ability of Monoclonal Antibody AwETN40a

A spiral strip about 2 cm long delivered from porcine left coronary artery was suspended in a Magnus tube filled with a Krebs-Henseleit solution (hereinafter referred to as nutritive solution) while passing a mixed gas (95% $O_2$ + 5% $CO_2$) therethrough and allowed to stand at 37°C for 3 hours. Then, the tension produced by the constriction of vascular smooth muscle was measured by an isometric transducer (polygraph, NEC Saneisha). When the solution ( with a final endothelin I concentration of 1 X $10^{-8}$ M) containing endothelin I previously reacted with 10-fold mole AwETN40a at 4°C for 3 hours was added to the muscle, the tension produced in the muscle was only 8.3 ± 3.6% (n = 4) of the tension produced therein by 60 mM KCl. In contrast, when the solution (with a final concentration of 1 X $10^{-8}$ M) containing endothelin I not reacted with AwETN40a was used, the tension approximately equivalent to that produced by 60 mM KCl (78 ± 30%, n = 4 was observed. From the fact described above, it was proved that AwETN40a neutralized the vascular smooth muscle constrictive activity of endothelin I.

Example 14

Competitive method - EIA

(1) EIA Using Polyclonal Antibody to Immunogen (I)

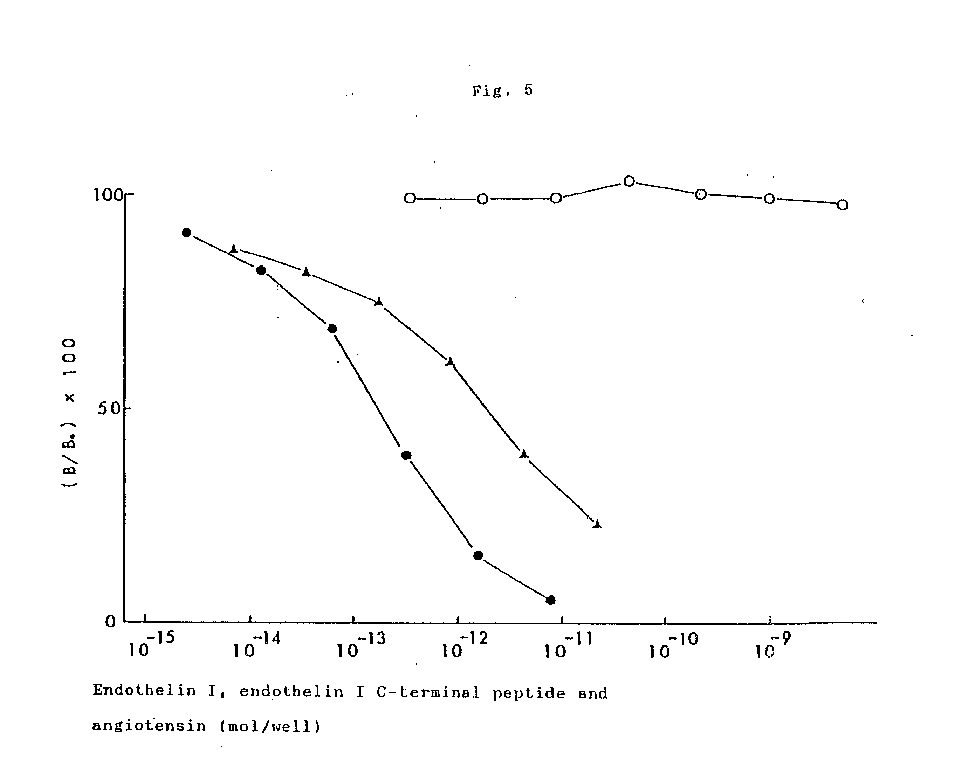

To an anti-rabbit IgG-bound microtiter plate, 50 $\mu$l of rabbit anti-endothelin serum No. 1a to immunogen (I) diluted finally 200,000 times with buffer A (refer to Fig. 1), and 50 $\mu$l of the standard solution of endothelin I, 50$\mu$l of the standard solution of polypeptide Cys His Leu Asp Ile Ile Trp or 50 $\mu$l of the standard solution of angiotensin-II (purchased from Peptide Laboratory) were added, and then the reaction was conducted at 4°C for 16 hours. Thereafter, 50 $\mu$l of peroxidase-labelled endothelin I (diluted 300 times with buffer A) was added thereto and the reaction was conducted at room temperature for 4 hours. After the reaction was completed, the resulting product was thoroughly washed with PBS. Then, the enzyme activity on the solid phase was measured by the ;method described above. The results are shown in Fig. 5. In the drawing, - ● -, - ▲ -and - o - indicate standard curves of endothelin I, polypeptide Cys His Leu Asp Ile Ile Trp and angiotensin-II, respectively. On the ordinate of the graph in Fig. 5, B represents the enzyme activity on the solid phase in the presence of the antigen such as endothelin I, angiotensin-II or the like, and $B_0$ represents the enzyme activity on the solid phase in the absence of the antigen.

It is anticipated that the conventional assays using the biological activity as a parameter will be influenced by another vasoconstrictor material such as angiotensin-II. However, the above results show that the immunoassay using the anti-endothelin antibody of the present invention are not influenced by angiotensin-II and can specifically measure endothelin I.

It was further found that this antiserum was capable of recognizing also endothelin I C-terminal polypeptide (Cys His Leu Asp Ile Ile Trp). Its affinity was about 1/10 of that to endothelin I. With respect to other rabbit antisera to immunogen (I), similar experiments were performed. Consequently, antibodies reactive to endothelin I C-terminal polypeptide were detected in all of these antisera. Their affinity was 1/2 to 1/10 of that to endothelin I.

(2) EIA Using Polyclonal Antibody to Immunogen (II)

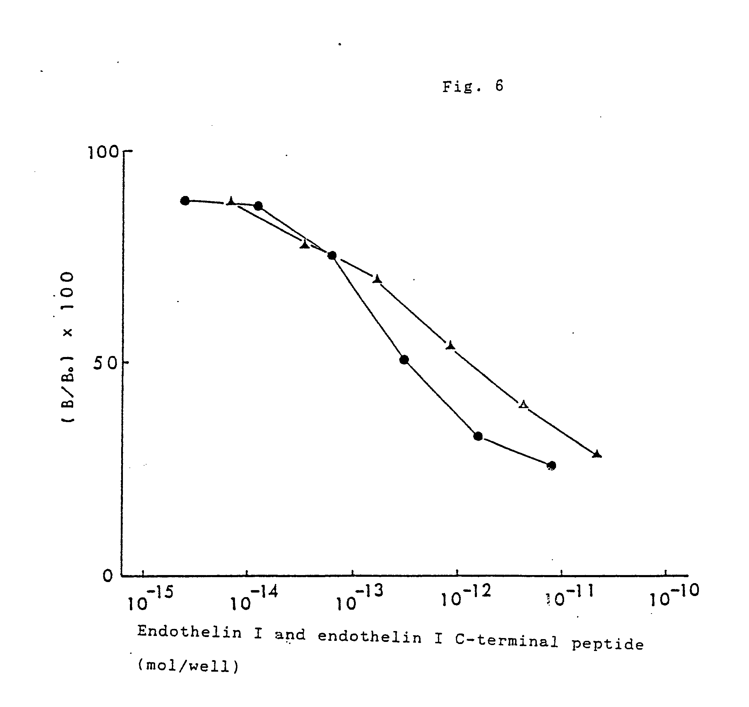

To an anti-rabbit IgG-bound microtiter plate, 50 $\mu$l of rabbit anti-endothelin serum No. 1c to immunogen (II) diluted finally 60,000 times with buffer A, and 50 $\mu$l of the standard solution of endothelin I or 50 $\mu$l of the standard solution of polypeptide Cys His Leu Asp Ile Ile Trp were added, and then te reaction was conducted at 4°C for 16 hours. Thereafter, 50 $\mu$l of peroxidase-labelled endothelin I (diluted 100 times with buffer A) was added thereto and the reaction was conducted at room temperature for 4 hours. After the reaction was completed, the resulting product was thoroughly washed with PBS. Then, the enzyme activity on the solid phase was measured by the method described above. The results are shown in Fig. 6. In the drawing, - ● - and - ▲ - indicate standard curves of endothelin I and polypeptide Cys His Leu Asp Ile Ile Trp, respectively. The above results showed that rabbit antiserum No. 1c prepared by immunizing endothelin I C-terminal polypeptide was capable of recognizing also endothelin I.

### (3) EIA Using Monoclonal Antibody AwETN40a

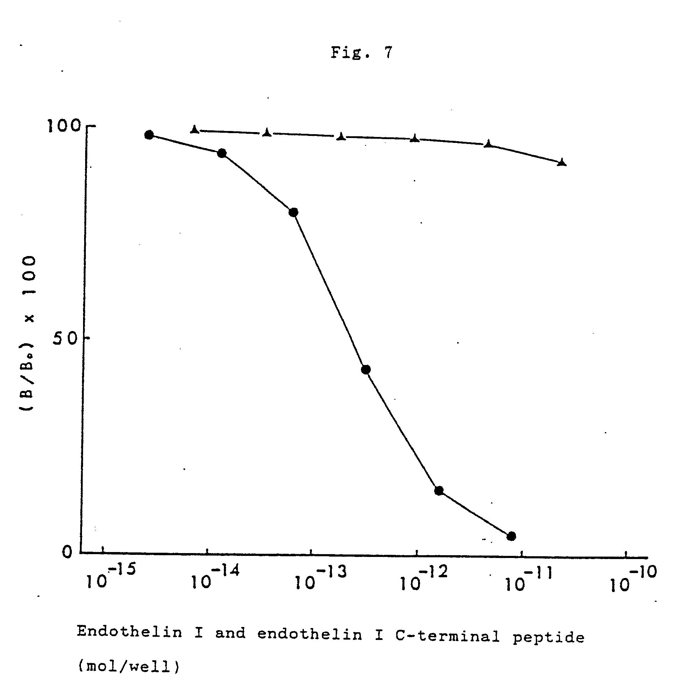

To an anti-mouse IgG-bound microtiter plate, 50 μl of a culture supernatant containing AwETN40a diluted finally 75 times with buffer A, and 50 μl of the standard solution of endothelin I or 50 μl of the standard solution of polypeptide Cys His Leu Asp Ile Ile Trp were added, and then the reaction was conducted at 4°C for 16 hours. After washing with PBS, 50 μl of peroxidase-labelled endothelin I (diluted 300 times with buffer A) was added thereto and the reaction was conducted at room temperature for 4 hours. After the reaction was completed, the resulting product was thoroughly washed with PBS. Then, the enzyme activity on the solid phase was measured by the method described above. The results are shown in Fig. 7. In the drawing, - ● - and - ▲ - indicate standard curves of endothelin I and polypeptide Cys His Leu Asp Ile Ile Trp, respectively.

From the results shown in Fig. 17, it was found that AwETN40a reacts wit endothelin I and does not react with endothelin I C-terminal polypeptide, namely that AwETN40a recognizes portions other than the C-terminal polypeptide portion of endothelin I.

### (4) EIA Using Polyclonal Antibody to Immunogen (III)

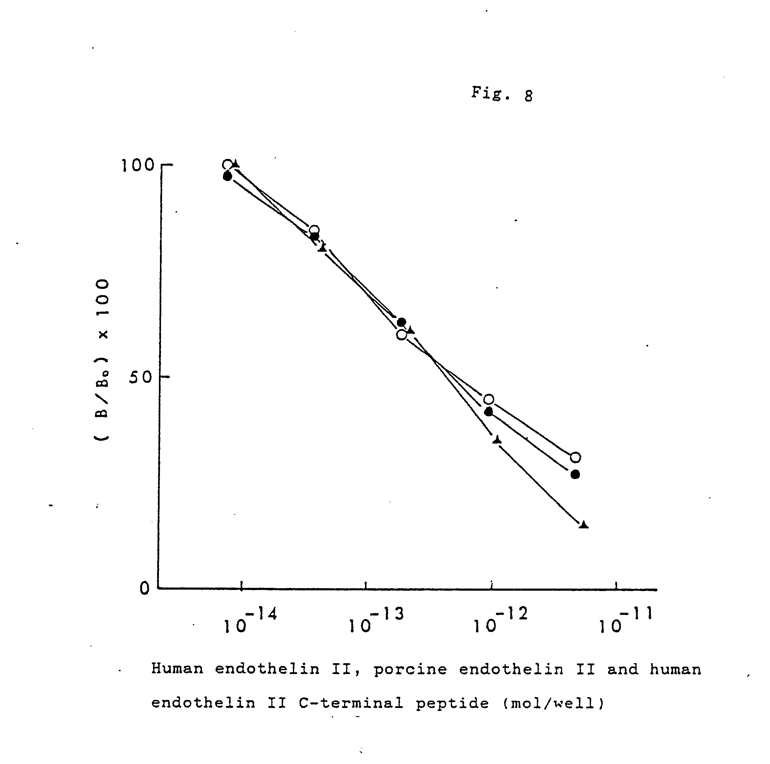

To an anti-rabbit IgG-bound microtiter plate, 50 μl of rabbit anti-endothelin serum No. 1b to immunogen (III) diluted finally 90,000 times with buffer A, and 50 μl of the standard solution of human endothelin II, 50 μl of the standard solution of porcine endothelin II or 50 μl of the standard solution of human endothelin II C-terminal peptide were added, and then the reaction was conducted at 4°C for 16 hours. Thereafter, 50μl of peroxidase-labelled endothelin II (diluted 600 times with buffer A) was added thereto and the reaction was conducted at room temperature for 4 hours. After the reaction was completed, the resulting product was thoroughly washed with PBS. Then, the enzyme activity on the solid phase was measured by the method described above. The results are shown in Fig. 8. In the drawing, - ● -, - o - and - ▲ - indicate standard curves of human endothelin II, porcine endothelin II and human endothelin II C-terminal peptide, respectively. The above results showed that rabbit antiserum No. 1b prepared by using human endothelin II C-terminal polypeptide as an immunogen had the reactivity equivalent to those of human endothelin II and porcine endothelin II.

### Example 15

### Sandwich Method-EIA (i)

The sandwich method-EIA for determining endothelin I will hereinafter be described.

### (1) Preparation of Enzyme-Labelled Antibody

A Fab'-peroxidase conjugate was prepared from the affinity-purified anti-Cys His Leu Asp Ile Ile Trp antibody described in (2) of Example 7 according to the method of Ishikawa et al. [J. Appl. Biochem., 6, 56 - 63 (1984)],

That is to say, 160 μg of pepsin (crystallized twice, Sigma) was added to 0.1 M acetate buffer (pH 4.5) in which 6.4 mg of the specific antibody was dissolved, and reacted therewith at 37°C for 16 hours. Then, the F(ab')2 fractions were purified by FPLC (Pharmacia) using a Superlose 12 column equilibrated with BBS. After those fractions were dialyzed against 0.1 M acetate buffer (pH 5), β-mercaptoethylamine was added to a final concentration of 20 mM and the resulting solution was allowed to stand at 37°C for 90 minutes. The reaction solution was separated by FPLC using a Superlose 12 column equilibrated with 0.1 M phosphate buffer (pH 6.0) containing 2.5 mM EDTA. Thus, the Fab' fraction was obtained.

On the other hand, 5 mg of horseradish peroxidase was dissolved in 0.9 ml of 0.1 M phosphate buffer (pH 7), and 1.05 mg of GMBS dissolved in 50 $\mu$l of DMF was added thereto. The reaction was conducted at room temperature for 40 minutes. The reaction solution was separated on a Sephadex G-25 column ( eluent: 0.1 M phosphate buffer, pH 6.8), and 3.5 mg of maleimidated peroxidase obtained thereby was mixed with 0.8 mg of the above Fab' fraction. The resulting mixture was condensed to about 0.3 ml by a collodion pak (Emuesu Instruments) and then allowed to stand at 4°C for 16 hours. The reaction solution was applied on a Ultrogel AcA44 column (10 mm $\phi$ X 40 mm) using 0.1 M phosphate buffer as an eluent to purify the Fab'-peroxidase conjugate fraction.

(2) Sandwich method-EIA (colorimetry)

Into each well of a 96-well microtiter plate, 100 $\mu$l portions of 0,1 M carbonate buffer (pH 9.,6) containing 20 $\mu$g/ml of purified monoclonal antibody AwETN40a were separately poured, and allowed to stand at 4°C for 24 hours. Excess binding sites of the wells were inactivated by adding 300 $\mu$l of Block Ace (manufactured by Snow Brand Milk Products, sold by Dainippon Pharmaceutical) diluted 4 times with PBS.

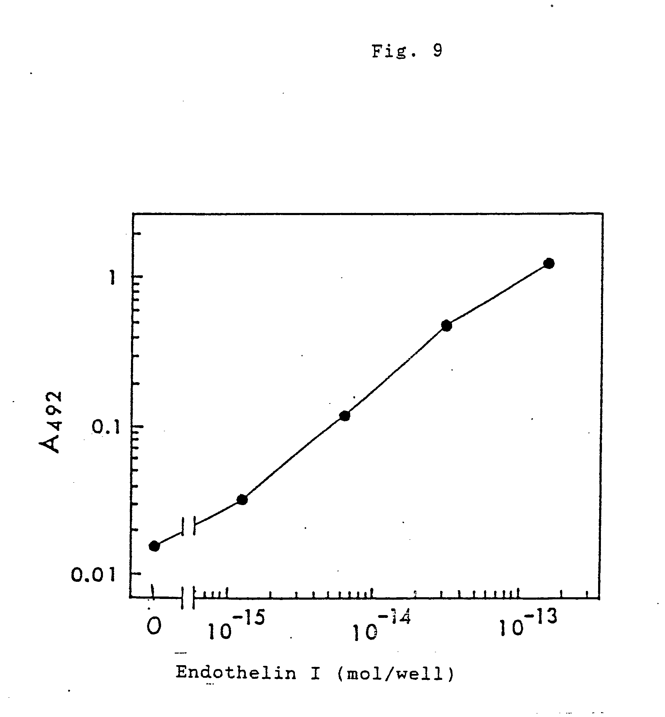

To the microtiter plate prepared as described above was added 100 $\mu$l of the endothelin I standard solution diluted with buffer A, and the reaction was conducted at room temperature for 5 hours. After the plate was washed with PBS, 100 $\mu$l of anti-Cys His Leu Asp Ile Ile Trp antibody Fab'-peroxidase conjugate (diluted 300 times with buffer A) was added thereto, and then the reaction was conducted at room temperature for 3 hours. After the plate was washed with PBS, the enzyme activity on the solid phase was assayed by the method described in Example 5. The results are shown in Fig. 9.

It was proved from the above results that 40 pg/ml(1.6 x10$^{-15}$ moles/well) of endothelin I could be determined by the sandwich method-EIA(colorimetry) in which anti-endothelin monoclonal antibody recognizing portions other than the endothelin I C-terminal portion was used as a solid phase, and polyclonal antibody Fab' recognizing the endothelin I C-terminal portion was used as an enzyme conjugate.

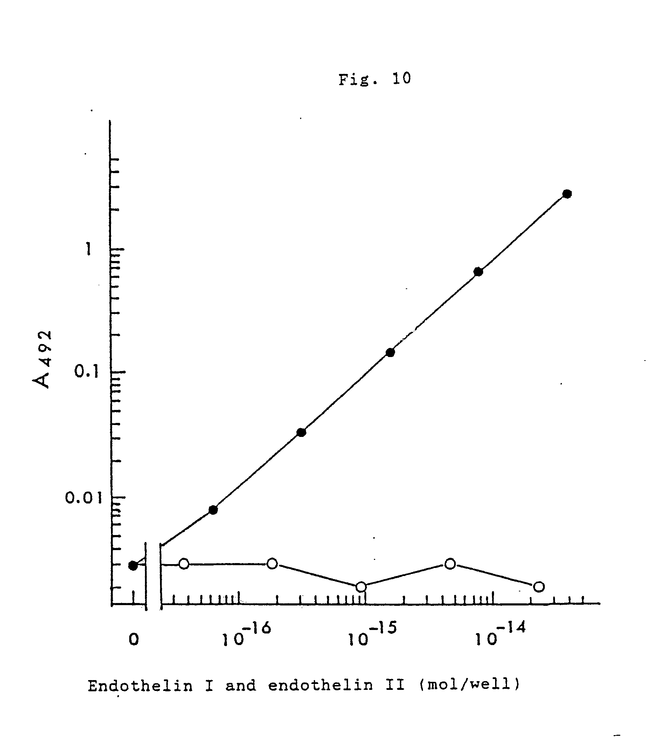

In order to obtain a higher assay sensitivity and examine the reactivity to endothelin II +, the following experiments were carried out. Namely, 100 $\mu$l of the standard solution of endothelin I or human endothelin II diluted with buffer E [0.02 M phosphate buffer (pH 7.2) containing 0.4 M NaCl, 2 mM EDTA, 0.2% BSA and 10% Block Ace) was added to the AwETN40a-fixed microtiter plate described above, and the reaction was conducted at room temperature for 24 hours. After the plate was washed, 100 $\mu$l of the anti-Cys His Leu Asp Ile Ile Trp antibody-peroxidase conjugate solution diluted 100 times with buffer C [0.02 M phosphate buffer (pH 7.2) containing 0.4 M NaCl, 2 mM EDTA and 1% BSA] was added thereto and then the reaction was conducted at 4°C for 16 hours. Thereafter, the enzyme activity on the solid phase was assayed by the method described in Example 5. The results are shown in Fig. 10.

In the drawing, - ● - and - o - indicate standard curves of endothelin I and endothelin II, respectively.

It was found from the above results that the extended reaction time up to 24 hours and the improvement of measuring conditions enabled the determination of 2 pg/ml (80 amoles/well) of endothelin I. It was also ascertained that this method was specific to endothelin I and unreactive to endothelin II in this assay.

Example 16

Sandwich Method-EIA (ii)

The sandwich method-EIA in which the antibody was positioned reverselt to the arrangement in the EIA described in Example 15 was carried out.

(1) Preparation of Enzyme-Labelled Antibody

Monoclonal antibody AwETN40a purified in Example 11 was labelled with peroxidase by the method described in Example 4.

That is to say, twenty times molar excess (182 $\mu$g) of GMBS was added to 33 nmoles of AwETN40a (5mg) in 0.1 M phosphate buffer (pH 6.7), and the reaction was conducted at room temperature for 40 minutes. After the reaction was completed, the reaction product was fractionated on a Sephadex G-25 column to obtain AwETN40a in which maleimide groups were introduced.

On the other hand, 10 mg (250 nmoles) of horseradish peroxidase was dissolved in 1.4 ml of 0.02 M phosphate buffer (pH 6.8) containing 0.15 M NaCl, and 100 $\mu$l of a DMF solution containing 1.17 mg (3.75 $\mu$ moles) of SPDP was mixed therewith. Then, the reaction was conducted at room temperature for 40 minutes. After the reaction was completed, 0.5 ml of 0.1 M acetate buffer (pH 4.5) containing 12.4 mg (80 $\mu$moles) of dithiothreitol was added thereto and the reaction was conducted at room temperature for 20 minutes. The resulting product was fractionated on a Sephadex G-25 column to obtain 6 mg (150 nmoles) of the enzyme in which SH groups were introduced. Thereafter, 20 nmoles of maleimide group-introduced AwETN40a and 100 nmoles of SH group-introduced peroxidase were mixed and reacted with each other at 4°C for 16 hours. After the reaction was completed, the reaction product was fractionated on a Ultrogel AcA34 column to obtain peroxidase-labelled AwETN40a.

(2) Sandwich Method-EIA

Into each well of a 96-well microtiter plate was separately poured 100 $\mu$l portions of 0.1 M carbonate buffer (pH 9.6) containing 20 $\mu$g/ml of the affinity-purified anti-Cys His Leu Asp Ile Ile Trp antibody [immunogen (II)] or the affinity-purified anti-endothelin I antibody [immunogen (I)] described in (2) of Example 7 and allowed to stand at 4°C for 24 hours. Excess binding sites of te wells were inactivated by adding 300 $\mu$l of Block Ace (manufactured by Snow Brand Milk Products, sold by Dainippon Pharmaceutical) diluted 4 times with PBS.

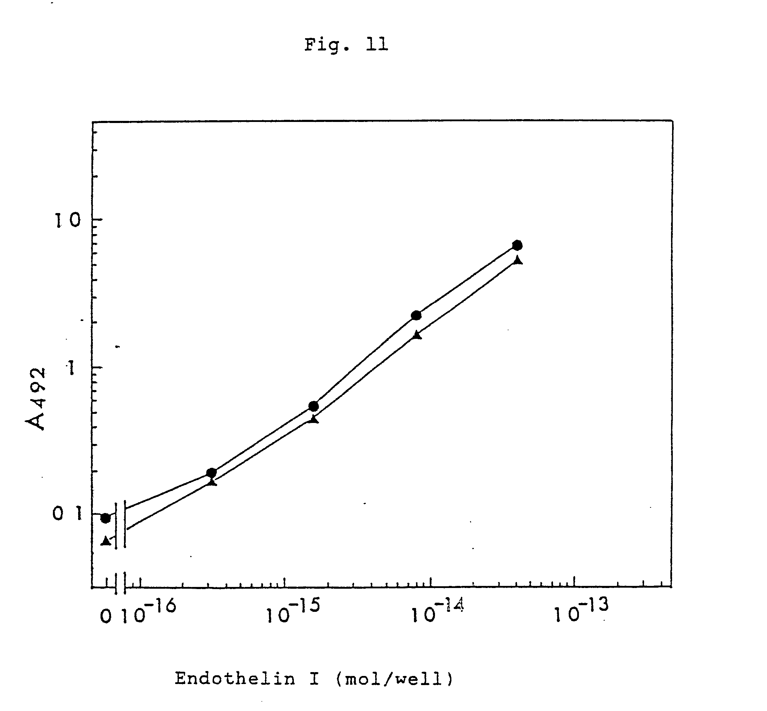

To the plate prepared as described above was added 100 $\mu$l of the standard solution of endothelin I diluted with buffer A, and the reaction was conducted at room temperature for 24 hours. After the plate was washed, 100 $\mu$l of peroxidase-labelled AwETN40a (diluted 5,000 times with buffer A) was added thereto, and then the reaction was conducted at 4°C for 16 hours. Thgereafter, the enzyme activity on the solid phase was assayed by the method described in Example 5. The results are shown in Fig. 11. In the drawing, - ● - indicates a standard curve of endothelin I when the anti-endothelin I antibody [immunogen (I)] purified on the Arg H is Leu Asp Ile Ile Trp-bound affinity column was used as an antibody for a solid phase, and - ▲ - indicates a standard curve thereof when the affinity-purified anti-Cys His Leu Asp Ile Ile Trp antibody [immunogen (II)] was used as an antibody for a solid phase.

It was found from the above results that 8 pg/ml (3.2 X $10^{-16}$ moles/well) of endothelin I could be assayed by the assay system in which the antibody was arranged reversely to that in Example 15, namely by the sandwich method-EIA in which the antibody recognizing the endothelin I C-terminal portion was used as a solid phase and anti-endothelin I antibody AwETN40a recognizing portions other than the endothelin I C-terminal portion was used as an enzyme conjugate.

Example 17

Sandwich method-EIA (iii)

The sandwich method-EIA for assay endothelin II will hereinafter be described.

(1) Preparation of Enzyme-Labelled Antibody

A Fab'-peroxidase conjugate was prepared form 12 mg of the affinity-purified anti-human endothelin II C-terminal peptide described in (3) of Example 7 according to the method of Ishikawa et al. [J. Appl. Biochem., 6, 56 - 63 (1984)].

(2) Sandwich method-EIA (colorimetry)

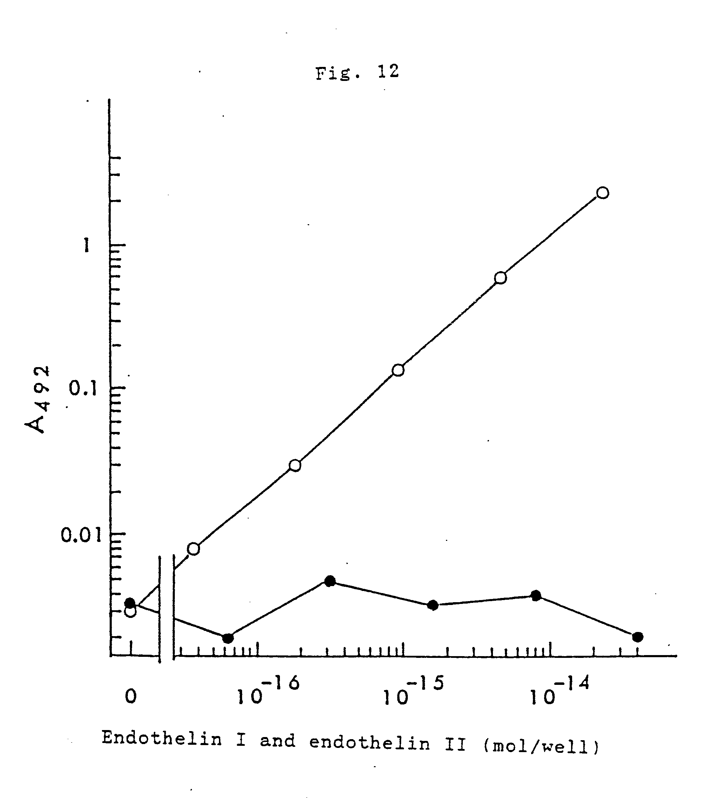

To the AwETN40a-fixed microtiter plate described in (2) of Example 15 was added 100 $\mu$l of the standard solution of endothelin I or endothelin II diluted with buffer A or 100 $\mu$l of the standard solution of human endothelin II, and the reaction was conducted at room temperature for 24 hours. After the plate was washed, 100 $\mu$l of the above-mentioned anti-endothelin II C-terminal peptide antibody Fab'-peroxidase conjugate (diluted 300 times with buffer A) was added thereto and then the reaction was conducted at 4°C for 16 hours. Thereafter, the enzyme activity on the solid phase was assayed by the method described in Example 5. The results were shown in Fig. 12.

In the drawing, - ● - and - o - indicate standard curves of endothelin I and endothelin II, respectively.

It was proved from the above results that down to about 4pg/ml (90 moles/well) of human endothelin II could be detected by this sandwich-EIA without the reaction with endothelin I.

The polyclonal antibody and the monoclonal antibody of the present invention have a high affinity for endothelin and advantageously used for detection or purification of endothelin or a part thereof by immunological procedures. These antibodies are particlularly useful in sandwich immunoassays in which each of two antibodies having an affinity for differing partial regions of endothelin are used whereby endothelin I and endothelin II can be selectively determined with very high sensitivity.

The affinity(Kd) of the antibodies of the present invention is from about $10^{-14}$ to $10^{-7}$ M. The Kd means dissociation constant.

## Example 18

Assay of Immuno Activity of endothelin I and endothelin II in human blood plasma

i) Human whole blood was collected from an elbow vein and 300 KIE/ml (a final concentration) of trypsine-inhibitor (Antagosan, Hechst) and 2 mg/ml of EDTA were immediately added thereto. The resulting solution was centrifuged at 1,500xg for 10 minutes to obtain a blood plasma. The plasma was stored at $-80°$ C.

ii) Pre-treatment of a Blood Plasma

4% acetic acid was added to the blood plasma to a final concentration of 3%, and the resulting solution was applied to a Sep-pak C18 cartridge (Waters) previously pre-treated in accordance with Scheme 1 which is outlined herein below. After washing the cartridge with distilled water and eluting the adsorbed fraction with 86% ethanol containing 4% acetic acid, the eluate was concentrated in a nitrogen stream at normal pressure to obtain a dried solid. The concentrate was dissolved in 250 $\mu$l of Buffer E and the indissoluble materials were removed by centrifugation. The resulting product was assayed by the Sandwich-method-EIA for endothelin I or endothelin II.

iii) Addition and recovery of endothelin I and endothelin II using blood plasma sample.

25 pg/ml, 60 pg/ml or 200 pg/ml of endothelin I or 50 pg/ml of human or porcine endothelin I + I was added to 0.5 to 1 ml of human blood plasma. After the blood plasma was pre-treated according ot the method described in above ii), it was assayed by sandwitch-method-EIA. The results are shown in Table 1 and Table 2. The recovery rates of endothelin I were over 80%, those of human and porcine endothelin II were 63% and 77% respectively. These results show that the pre-treatment of blood plasma is proper.

iv) Assay of Immuno Activity of endothelin I and endothelin II in normal human blood plasma

Table 3 shows average level of endothelin I(abbreviated at ET in the table) and endothelin II (abbreviated as Big-ET in the table) in blood plasma of twenty four men and twenty three women. In blood, the level of endothelin II was about two times that of endothelin I.

v) Change of Blood Plasma Endothelin I or Myocardial Infarct Patient

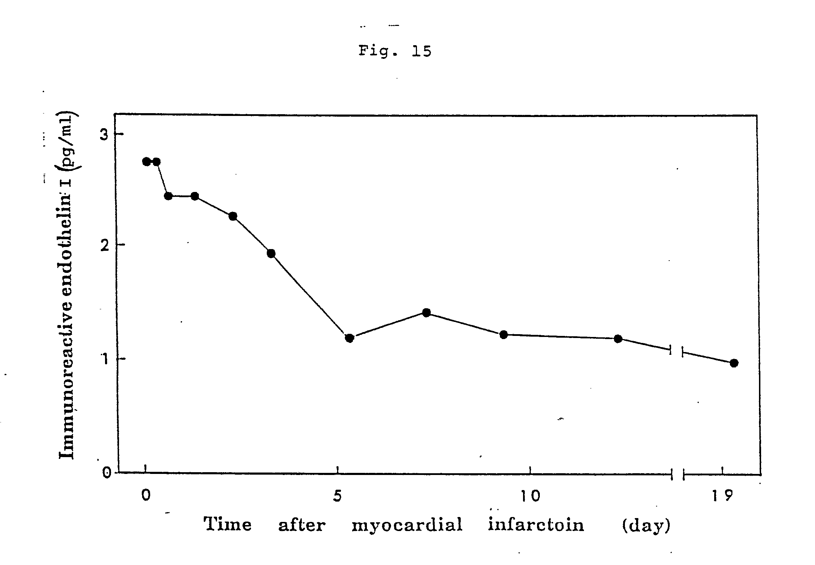

Level of blood plasma endothelin of male myocardial infarct patient 67 years old was assayed (Fig. 15).

From immediately after the symptoms of the disease, the level of endothelin I in blood showed high value and was gradually reduced to a normal level. This result shows that the convalescant control of a myocardial infarct patient can be conducted by the assay of endothelin level in blood.

It is understood that the examples and embodiments described herein are for illustrative purposes only, and that various modifications or changes in light thereof that will be suggested to persons skilled in the art are to be included in the spirit and purview of this application and the scope of the approved claims.

Pretreatment of Seppak C-18 cartridges with:

-5 ml of 4% acetic acid in 86% ethanol

-5 ml of methanol

-5 ml of distilled water

-5 ml of 4% acetic acid

Acidification of plasma (0.5 or 1.0 ml) with 3 ml of 4% acetic acid

Loading to the cartridges

Washing with 10 ml of distilled water

Eluting with 4 ml of 4% acetic acid in 86% ethanol

Evaporation under a stream of nitrogen gas at 35°C

Reconstitution with 0.25 ml of buffer E
↓
Sandwich-EIA

Scheme . Procedures for the extraction of endothelin in plasma[1]. 1) Rosmalen F. et al. (1987) Clin. Chim. Acta, 331-340.

Table 1

| Recovery of endothelin added to plasma samples | | | | |
|---|---|---|---|---|
| Exp. No. | Endothelin added (pg/ml)[1] | No. of plasma tested (n) | Endothelin recovered | |
| | | | (pg/ml) | (%) |
| 1 | 25 | 14 | 20.2±1.3[2] | 80.6±5.3 |
| 2 | 60 | 4 | 53.6±3.5 | 90.0±5.8 |
| 3 | 200 | 4 | 184±9.0 | 92.0±4.5 |

1)Endothelin at 25, 30, and 100 pg/ml was added to 1, 0.5, and 0.5 ml of plasma, respectively, and acidified with 3 ml of 4% acetic acid. The solutions were applied to Seppak C18 cartridges and the adsorbed fractions were subjected to the sandwich-EIA.
2) Mean ± SD

EP 0 331 100 A1

Table 2

| Recovery of big-endothelin added to human plasma | | | | |
|---|---|---|---|---|
| Big-ET | Big-ET added (pg/ml)[1] | No. of Plasma tested (n) | Big-ET recovered | |
| | | | (pg/ml) | (%) |
| Human | 50 | 4 | 31.5±4.5 | 63.0±8.9 |
| Porcine | 50 | 4 | 38.3±2.3 | 76.5±4.6 |

1)Big-ET at 50 pg/ml was added to 1.0 ml of plasma, and acidified with 3 ml of 4% acetic acid. The solutions were applied to Seppak C-18 cartridges and the adsorbed fractions were subjected to the sandwich-EIA.

2) Mean ± SD

Table 3

| Immunoreactive endothelin and big-endothelin in human peripheral venous plasma. | | | | |
|---|---|---|---|---|
| | Age | No.of plasma | Plasma immunoreactive ETs | |
| | | | Big-ET (pg/ml) | ET (pg/ml) |
| Male | 31-49 | 24 | 3.28±0.59[1] | 1.63±0.48 |
| Famale | 22-55 | 23 | 3.64±1.03 | 1.53±0.50 |

1) Mean±SD

**Claims**

1. A polyclonal antibody having an affinity for endothelin.

2, A polyclonal antibody according to claim 1, wherein the affinity(Kd) is from about $10^{-14}$ to about $10^{-7}$ M.

3. A polyclonal antibody according to claim 1, wherein the antibody recognized the peptide represented by the following amino acid sequence: Cys Ser Cys Ser Ser Leu Met Asp Lys Glu Cys Val Tyr Phe Cys His Leu Asp Ile Ile Trp

4. A polyclonal antibody according to claim 1, wherein the antibody recognizes the peptide represented by the following amino acid sequence:
Cys Ser Cys Ser Ser Leu Met Asp Lys Glu Cys Val Tyr Phe Cys His Leu Asp Ile Ile Trp Val Asn Thr Pro Glu His Val Val Pro Tyr Gly Leu Gly Ser Pro Arg Ser

5. A polyclonal antibody according to claim 1, wherein the antibody recognizes the peptide represented by the following amino acid sequence: Cys Ser Cys Ser Ser Leu Met Asp Lys Glu Cys Val Tyr Phe Cys His Leu Asp Ile Ile Trp Val Asn Thr Pro Glu His Val Val Pro Tyr Gly Leu Gly Ser Pro Ser Arg Ser

6. A polyclonal antibody according to claim 1, wherein the antibody recognizes the peptide represented by Cys His Leu Asp Ile Ile Trp.

7. A polyclonal antibody according to claim 1, wherein the antibody recognizes the peptide represented by Val Asn Thr Pro Glu His Val Val Pro Tyr Gly Leu Gly Ser Pro Arg Ser.

8. A polyclonal antibody according to claim 1, wherein the antibody recognizes the peptide represented by Val Asn Thr Pro Glu His Ile Val Pro Tyr Gly Leu Gly Ser Pro Ser Arg Ser.

9. A monoclonal antibody having an affinity for endothelin.

25

10. A monoclonal antibody according to claim 9, wherein the affinity(Kd) is from about $10^{-14}$ to about $10^{-7}$ M.

11. A monoclonal antibody according to claim 9, which has an affinity for endothelin and binds to endothelin or a fragment thereof at an amino acid sequence other than Cys His Leu Asp Ile Ile Trp.

12. A monoclonal antibody according to claim 9, which has an affinity for endothelin and binds to endothelin or a fragment thereof at an amino acid sequence other than Val Asn Thr Pro Glu His Val Val Pro Tyr Gly Leu Gly Ser Pro Arg Ser, or
Val Asn Thr Pro Glu His Ile Val Pro Tyr Gly Leu Gly Ser Pro Ser Arg Ser.

13. A hybridoma which produces a monoclonal antibody to endothelin.

14. Hybridoma cell AwETN40.

15. A monoclonal antibody produced by hybridoma AwETN40.

16. A method for determining the amount of endothelin in a test solution wich comprises competitively reacting an anti-endothelin antibody with the test solution and a known amount of labelled endothelin, and determining the amount of the labelled endothelin bound to said antibody.

17. A method for determining the amount of endothelin in a test solution which comprises the steps of (a) contacting the solution with first antibody to endothelin insolubilized on a carrier, (b) contacting a second labelled antibody to endothelin therewith, and (c) determining the amount of labelled second antibody which is immobilized.

18. A method according to claim 17, wherein one of the antibodies is a monoclonal antibody produced by hybridoma AwETN40.

19. A method according to claim 17, wherein one of the antibodies is a polyclonal antibody which recognizes a C-terminal peptide sequence of the endothelin, and the other antibody is a monoclonal antibody which recognizes a peptide sequence of endothelin different from that recognized by the polyclonal antibody.

20. A method according to claim 19, wherein the monoclonal antibody recognizes a peptide sequence of endothelin other than a C-terminal peptide sequence.

21. A method according to claim 19, wherein the monoclonal antibody is one produced by hybridoma AwETN40.

Fig. 1

Fig. 2

Fig. 3

Fig. 4

Antiserum dilution    $(\times 10^{2})$

Fig. 5

$(B/B_0) \times 100$

Endothelin I, endothelin I C-terminal peptide and angiotensin (mol/well)

Fig. 6

Endothelin I and endothelin I C-terminal peptide
(mol/well)

Fig. 7

Endothelin I and endothelin I C-terminal peptide
(mol/well)

Fig. 8

Human endothelin II, porcine endothelin II and human endothelin II C-terminal peptide (mol/well)

Fig. 9

Endothelin I (mol/well)

Fig. 10

Endothelin I and endothelin II (mol/well)

Fig. 11

Endothelin I (mol/well)

Fig. 12

Endothelin I and endothelin II (mol/well)

Fig. 13-1

CATTTTTCTGCCTTTTTCCCCCGCTTTCGGTTTAGTTCGCAGGGGGAGGATTTTGATCCTTTTTTTTTTTCAGA

ATG GAT TAT TTC CCC ATG ATT ATC GCT CTG CTG TTT GTG GCT TTC CAA GGA GCT CCA GAA
Met Asp Tyr Phe Pro Met Ile Ile Ala Leu Leu Phe Val Ala Phe Gln Gly Ala Pro Glu

ACA GCG GTC CTG GGC GCC GAG CTC AGC CCG GAA GCC GAG AGC CAA GGG GAG ACG CCC TCT
Thr Ala Val Leu Gly Ala Glu Leu Ser Pro Glu Ala Glu Ser Gln Gly Glu Thr Pro Ser

CCC CAT GCA TCC TGG AGG CCC CGT CGG TCC AAG CGC TGC TCC TGC TCT TCC CTG ATG GAT
Pro His Ala Ser Trp Arg Pro Arg Arg Ser Lys Arg Cys Ser Cys Ser Ser Leu Met Asp

AAA GAG TGT GTC TAC TTC TGC CAC CTG GAC ATC ATC TGG GTC AAC ACT CCA GAA CAC ATT
Lys Glu Cys Val Tyr Phe Cys His Leu Asp Ile Ile Trp Val Asn Thr Pro Glu His Ile

GTC CCA TAC GGA CTT GGA AGC CCT TCT AGG TCC AGG CGA TCC TTA AAG GAT TTG TTT CCT
Val Pro Tyr Gly Leu Gly Ser Pro Ser Arg Ser Arg Arg Ser Leu Lys Asp Leu Phe Pro

EP 0 331 100 A1

Fig. 13-2

GCA AAG GCA GCA GAC CGC AGG GAT AGA TGC CAG TGT GCC AGC CAA AAA GAC AAG AAG TGC    360
Ala Lys Ala Ala Asp Arg Arg Asp Arg Cys Gln Cys Ala Ser Gln Lys Asp Lys Lys Cys   120


TGG AGT TTC TGC CAA GCA GGA AAA GAA ATC GGC AGG GAC CAA GAC ACA ATG GAG AAA CGC    420
Trp Ser Phe Cys Gln Ala Gly Lys Glu Ile Gly Arg Asp Gln Asp Thr Met Glu Lys Arg   140


TGG GAT AAC CAA AAG AAA GGA ACA GAC TGT TCC AAG CTT GGA GAG AAG TGT ATT CAT CGG    480
Trp Asp Asn Gln Lys Lys Gly Thr Asp Cys Ser Lys Leu Gly Glu Lys Cys Ile His Arg   160


CAG CTG GTG ATG GGA AGA AAA ATA AGA AGG TTG GAG GCC ATC AGC AAC AGC ATC AAA ACA    540
Gln Leu Val Met Gly Arg Lys Ile Arg Arg Leu Glu Ala Ile Ser Asn Ser Ile Lys Thr   180


TCT TTT CAC ATC GCC AAG CTG AAA GCC GAG CTC TAC AGA GAT AAG AAA GTG ACC CAT AAC    600
Ser Phe His Ile Ala Lys Leu Lys Ala Glu Leu Tyr Arg Asp Lys Lys Val Thr His Asn   200


CGA ACA CAC TGA TGACAGGTTGGCCGAGCCTGTCTGAAGCCATCTCGTCCCCAGGGAGCCCCGGGCCCGACTCTG    675
Arg Thr His ***                                                                    203

Fig. 14-1

```
                                                                        47
GA  ATT CGG GCT GCG CAG TCC GGG AAC GCG CAG CGC GCT TCC TGC AGT CCC

                                                                        95
AGC TCT CCA CCG CCG CCT CTC GAC TGC CTC TTC                             96

                                                                       143
TCT CCT GGC AGG CGC TGC TTA CCG TCC GCT ACT TGG GCT                    144

                                                                       191
GAA GGA TCG CTT GAT CTT TGC AAC CCG CAG CGC AAG GGA CCT                192

                                                                       239
GAA GCT GTT TTT CGT TTT CTT GGT TCA GTT TGA ACG GGA GGT               240

                                                                   287   9
TTT TGA TCC CTT CGT TTT TTC AGA ATG GAT TAT TTG CTC ATT TTC TCT       288  10
                              Met Asp Tyr Leu Leu Ile Phe Ser

                                                                   335  25
CTG CTG TTT GTG GCT TGC CAA GAA GGA GCT CCA ACA GCA GTC TTA GGC       336  26
Leu Leu Phe Val Ala Cys Gln Glu Gly Ala Pro Thr Ala Val Leu Gly

                                                                   383  41
GCT GAG CTC AGC GCG GTG GGT GAG GAG AAA GGG GGG AAC CCC ACT CCC       384  42
Ala Glu Leu Ser Ala Val Gly Glu Glu Lys Gly Gly Asn Pro Thr Pro

                                                                   431  57
AGT CCA CCC TGG CGG CTC CGG TCC CGC AAG │TGC TCC TCG│               432  58
Ser Pro Pro Trp Arg Leu Arg Ser Arg Lys │Cys Ser Ser│

                                                                   479  73
CTG ATG GAT GAG AAA TAC TGT TTC CAC CTG │ATC ATT TGG│              480  74
Leu Met Asp Glu Lys Tyr Cys Phe His Leu │Ile Ile Trp│

                                                                   527  89
GTC AAC ACT CCC GAG CAC GTT TAT GGA CTT CCT AGC CCT AGG              528  90
Val Asn Thr Pro Glu His Val Tyr Gly Leu Pro Ser Pro Arg

                                                                   575 105
TCC AAG AGA TTA GAG CTT TTA CTT CCC ACA GCA AAG GAC CGT              576 106
Ser Lys Arg Leu Glu Leu Leu Leu Pro Thr Ala Lys Asp Arg

                                                                   623 121
GAG AAT AGA CAA GCT CAA AGC AAA GAC AAG TGG TGG AAT               624 122
Glu Asn Arg Gln Ala Gln Ser Lys Asp Lys Trp Cys Asn

                                                                   671 137
TTT TGC CAA GCA GCA GAA AAA CTC GAA GCT ATT ATG GAC GAG AAA
Phe Cys Gln Ala Ala Glu Lys Leu Glu Ala Ile Met Asp Glu Lys
```

Fig. 14-2

```
672                                                                                          719
138  GAC  TGG  AAT  CAT  AAG  AAA  GGA  AAA  GAC  TGT  TCC  AAG  CTT  GGG  AAA              153
     Asp  Trp  Asn  His  Lys  Lys  Gly  Lys  Asp  Cys  Ser  Lys  Leu  Gly  Lys

720                                                                                          767
154  AAG  TGT  ATT  TAT  CAG  TTA  GTG  AGA  GGA  AGA  AAA  ATC  AGA  AGA  AGT              169
     Lys  Cys  Ile  Tyr  Gln  Leu  Val  Arg  Gly  Arg  Lys  Ile  Arg  Arg  Ser

768                                                                                          815
170  TCA  GAG  CAA  CAC  AGA  CAA  ACC  AGG  TCG  AGA  ACC  ATG  AGA  AAC  AGC              185
     Ser  Glu  Gln  His  Arg  Gln  Thr  Arg  Ser  Arg  Thr  Met  Arg  Asn  Ser

816                                                                                          863
186  GTC  AAA  TCA  TTT  CAT  GAT  CCC  AAG  CTG  CCC  GGC  AAG  CCC  TCC  AGA              201
     Val  Lys  Ser  Phe  His  Asp  Pro  Lys  Leu  Pro  Gly  Lys  Pro  Ser  Arg

864                                                                                          911
202  GAG  CGT  TAT  GTG  ACC  AAC  CGA  GCA  CAT  TGG  TGA  CAG  ACT  TCG  GGG              212
     Glu  Arg  Tyr  Val  Thr  Asn  Arg  Ala  His  Trp  ***

912                                                                                          959
     CCT  GTC  TGA  AGC  CAT  CTC  CAC  GAG  CCC  TGT  GGC  GGC  CGA  CTC  TGC
          *    *

960                                                                                         1007
     ACT  CTC  CAC  CCT  GGC  TGG  GAT  CAG  AGC  ATC  CTC  TGC  TGG  TTC  TTC

1008                                                                                        1055
     CTG  ACT  GGC  AAA  GGA  TTC  CCA  AAA  TTC  ACA  TTC  CAA  GAA  AGG  AGG

1056                                                                                        1103
     TTA  AGG  AGT  TCC  CCC  TGG  CTT  CAC  TGG  GTG  TCA  GTG  GTA  ACT  ACT

1104                                                                                        1151
     GCT  TTG  GTC  TCT  TCT  ATC  TGG  GGA  TGA  ACC  TGG  ACC  TCT  CAG  CAG
                                              *
                                              *
                                              *

1152
     AAA  CAC  ACA  GTC  ACA  TTC  GAA  TTC
```

Fig. 15

Time after myocardial infarctoin (day)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| T | JOURNAL OF IMMUNOLOGICAL METHODS, vol. 118, 31st March 1989, pages 245-250, Elsevier Science Publishers B.V.; N. SUZUKI et al.: "A sensitive sandwich-enzyme immunoassay for human endothelin" | | C 12 P 21/00<br>G 01 N 33/577<br>C 12 N 5/00 |
| A,D | NATURE, vol. 323, 31st March 1988, pages 411-415; M. YANAGISAWA et al.: "A novel potent vasoconstrictor peptide produced by vascular endothelial cells" | | |

TECHNICAL FIELDS
SEARCHED (Int. Cl.4)

C 12 P

The present search report has been drawn up for all claims

| Place of search | Date of completion of the search | Examiner |
|---|---|---|
| THE HAGUE | 02-06-1989 | TURMO Y BLANCO C.E. |